(19) Europäisches Patentamt
European Patent Office
Office européen des brevets

(11) **EP 4 741 487 A1**

(12) **EUROPEAN PATENT APPLICATION**
published in accordance with Art. 153(4) EPC

(43) Date of publication:
**13.05.2026 Bulletin 2026/20**

(21) Application number: **24859186.9**

(22) Date of filing: **10.07.2024**

(51) International Patent Classification (IPC):
**C12M 1/34** (2006.01)    **G01N 21/80** (2006.01)
**G01N 21/359** (2014.01)

(52) Cooperative Patent Classification (CPC):
**C12M 1/34; G01N 21/3577; G01N 21/359;
G01N 21/80**

(86) International application number:
**PCT/JP2024/024986**

(87) International publication number:
**WO 2025/047141 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.08.2023 JP 2023140911**

(71) Applicant: **Chitose Laboratory Corp.
Kawasaki-shi, Kanagawa 213-0012 (JP)**

(72) Inventors:
• **TAKASHIMA, Masatoshi
  Kawasaki-shi, Kanagawa 213-0012 (JP)**

• **HORII, Shumpei
  Kawasaki-shi, Kanagawa 213-0012 (JP)**
• **SAKAGUCHI, Kohei
  Kawasaki-shi, Kanagawa 213-0012 (JP)**
• **OKADA, Katsunori
  Kawasaki-shi, Kanagawa 213-0012 (JP)**
• **SUZUKA, Satoshi
  Kawasaki-shi, Kanagawa 213-0012 (JP)**
• **KAWAI, Tetsushi
  Kawasaki-shi, Kanagawa 213-0012 (JP)**

(74) Representative: **J A Kemp LLP
80 Turnmill Street
London EC1M 5QU (GB)**

(54) **DATA EXTRACTION SYSTEM, DATA EXTRACTION METHOD, AND DATA EXTRACTION PROGRAM**

(57)    This data extraction system includes: a setting unit that sets an input criterion indicating performance required for data analysis; an input unit that acquires an input signal indicating a measurement result of a measurement object in bioproduction; a preprocessing unit that performs a preprocessing operation on the input signal for comparison with the input criterion to generate a preprocessed signal; a postprocessing unit that, when the preprocessed signal does not satisfy the input criterion, performs a postprocessing operation using another signal obtained by measuring the measurement object on the preprocessed signal, and generates a postprocessed signal; and an extraction unit that extracts the preprocessed signal as extracted data when the preprocessed signal satisfies the input criterion, and extracts the postprocessed signal as extracted data when the postprocessed signal satisfies the input criterion.

*Fig.1*

## Description

## Technical Field

The present disclosure relates to a data extraction system, a data extraction method, and a data extraction program.

## Background Art

A technique for measuring a temporal change of a substance by a sensor is known. Patent Document 1 discloses a technique for detecting presence or absence of abnormality of a culture medium. The technique described in Patent Document 1 obtains a state of distribution of characteristic values of the culture medium, and sets a threshold value for abnormality determination based on the state of distribution. Patent Document 2 discloses a technique for analyzing deterioration of plastic materials and the like. The technique described in Patent Document 2 performs second-order differential processing on near-infrared spectrum data of plastic materials and the like obtained by near-infrared spectroscopy to obtain corrected spectrum data.

## Citation List

## Patent Literature

[Patent Literature 1] Japanese Patent No. 6977977
[Patent Literature 2] Japanese Unexamined Patent Publication No. 2022-7236

## Summary of Invention

## Technical Problem

It is desired to improve quality of data used for analysis.

## Solution to Problem

A data extraction system according to one aspect of the present disclosure includes: a setting unit that sets an input criterion indicating performance required for data analysis; an input unit that acquires an input signal indicating a measurement result of a measurement object in bioproduction; a preprocessing unit that performs a preprocessing operation on the input signal for comparison with the input criterion to generate a preprocessed signal; a postprocessing unit that performs a postprocessing operation using another signal indicating the measurement result of the measurement object on the preprocessed signal when the preprocessed signal does not satisfy the input criterion, to generate a postprocessed signal; and an extraction unit that extracts the preprocessed signal as extracted data when the preprocessed signal satisfies the input criterion, and extracts the postprocessed signal as extracted data when the postprocessed signal satisfies the input criterion.

In the data extraction system according to one aspect of the present disclosure, the preprocessing operation is performed on the input signal to generate the preprocessed signal. Then, the preprocessed signal and the input criterion indicating performance required for data analysis are compared. When the preprocessed signal satisfies the input criterion, the preprocessed signal is extracted as the extracted data. On the other hand, when the preprocessed signal does not satisfy the input criterion, the postprocessing operation is performed on the preprocessed signal to generate the postprocessed signal. Then, the postprocessed signal and the input criterion are compared. When the postprocessed signal satisfies the input criterion, the postprocessed signal is extracted as the extracted data. Thereby, the extracted data whose quality is ensured can be extracted. As a result, the quality of data used for analysis can be improved.

## Advantageous Effects of Invention

According to the present disclosure, it is possible to provide a technique capable of improving quality of data used for analysis.

## Brief Description of Drawings

FIG. 1 is a diagram schematically showing a configuration of a data extraction system according to a first embodiment.
FIG. 2 is a schematic diagram showing an example of operation of the data extraction system.
FIG. 3 is a diagram showing an example of optical spectroscopic measurement in bioproduction.
FIGS. 4(a) to 4(c) are graphs exemplifying extraction of a change in organic molecule concentration in near-infrared spectroscopic measurement.
FIG. 5 is a schematic diagram showing another example of operation of the data extraction system.
FIG. 6 is a schematic diagram showing still another example of operation of the data extraction system.
FIGS. 7(a) to 7(d) are graphs showing an example for explaining an effect by the preprocessing operation and setting of the input criterion.
FIGS. 8(a) to 8(d) are graphs showing another example for explaining an effect by the preprocessing operation and setting of the input criterion.
FIG. 9 is an overall diagram showing an example of application of the data extraction system according to an embodiment.
FIG. 10 is a diagram showing an example of overall operation of a bioproduction system including the data extraction system.

FIG. 11 is a diagram showing an example of operation of the data extraction system.

FIG. 12 is a diagram showing an example of hardware configuration related to the data extraction system.

**Description of Embodiment**

Hereinafter, embodiments of the present disclosure will be described with reference to the drawings. In the description of the drawings, same reference numerals are assigned to same elements, and overlapping description is omitted.

[Overview]

The data extraction system of the present disclosure extracts data measured by a sensor for data analysis. Hereinafter, data used for data analysis is referred to as "extracted data". In data analysis, minimum performance may be required for the extracted data. Hereinafter, the minimum performance is referred to as "evaluation index". The data extraction system can be applied to data analysis in, for example, bioproduction or pharmaceutical manufacturing, but is not limited thereto. In the present disclosure, an example in which the data extraction system is applied to data analysis in bioproduction will be described.

The data extraction system acquires a signal measured by a sensor as an input signal. The data extraction system sets an index of the input signal corresponding to the evaluation index as an input criterion. The data extraction system performs a preprocessing operation on the input signal for comparison with the input criterion to generate a preprocessed signal. The data extraction system compares the preprocessed signal with the input criterion to determine whether or not the input criterion is satisfied.

The data extraction system performs a postprocessing operation using another signal measured by the sensor on the preprocessed signal that does not satisfy the input criterion, to generate a postprocessed signal. The other signal may be, for example, a signal before or after the input signal in time series. The data extraction system compares the postprocessed signal with the input criterion to determine whether or not the input criterion is satisfied. Then, the data extraction system extracts the preprocessed signal or the postprocessed signal that satisfies the input criterion as the extracted data. The data extraction system may remove the postprocessed signal that does not satisfy the input criterion. The data extraction system may extract data obtained by performing an operation for output or the like on the preprocessed signal or the postprocessed signal as the extracted data. In this manner, the data extraction system extracts the extracted data that satisfies the evaluation index.

[Embodiment]

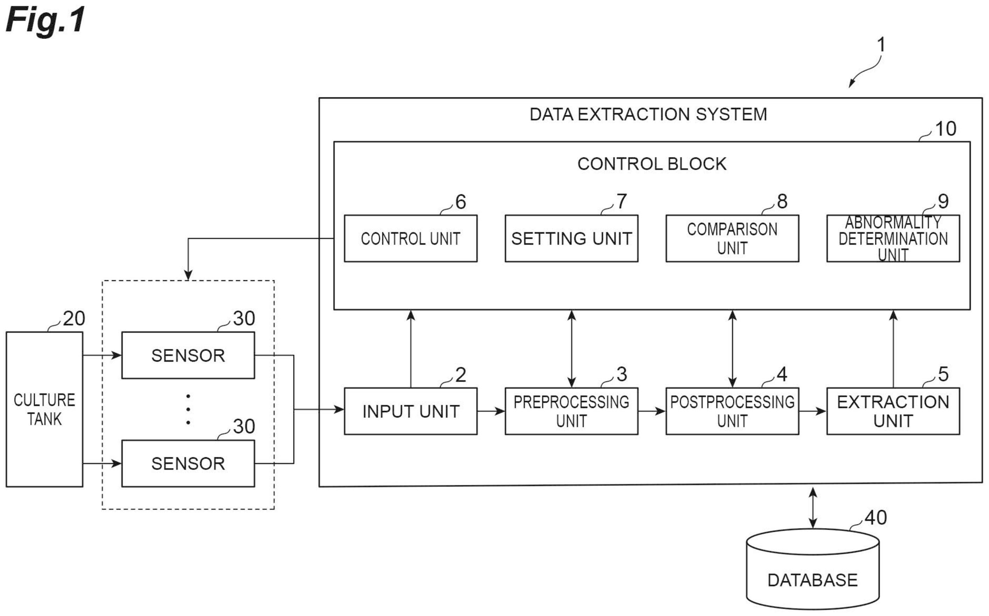

FIG. 1 is a diagram schematically showing a configuration of a data extraction system 1 according to an embodiment. The data extraction system 1 is communicably connected to a sensor 30 and a database 40. The data extraction system 1 acquires an input signal from, for example, the sensor 30, and extracts a plurality of pieces of extracted data. The data extraction system 1 stores the plurality of pieces of extracted data in the database 40.

The sensor 30 is a measurement device that outputs a signal indicating a measurement result of a measurement object in bioproduction. The number of sensors 30 is not limited. The sensor 30 is, for example, a hydrogen ion concentration (pH: Potential Hydrogen) sensor, a dissolved oxygen (DO: Dissolved Oxygen) sensor, a temperature sensor, a near-infrared sensor, an RGB sensor, a potential sensor, and a torque sensor, but is not limited thereto. The sensor 30 is installed in a culture tank 20. The culture tank 20 is a container used for cell culture or the like. The culture tank 20 contains a culture medium. The sensor 30 outputs a signal indicating a result of measurement of a state of the culture tank 20 to the data extraction system 1.

The database 40 is a non-transitory storage medium or storage device that stores information. The database 40 stores, for example, the evaluation index and the extracted data. The database 40 may store a signal acquired by the sensor 30 as data. The database 40 may be constructed as a single database or may be a set of a plurality of databases. The installation location of the database 40 is not limited. For example, the database 40 may be provided in a computer system different from the data extraction system 1.

The data extraction system 1 includes, as functional elements, an input unit 2, a preprocessing unit 3, a postprocessing unit 4, an extraction unit 5, and a control block 10. The control block 10 includes, as functional elements, a control unit 6, a setting unit 7, a comparison unit 8, and an abnormality determination unit 9.

The input unit 2 acquires a signal output from the sensor 30 as an input signal. The input unit 2 acquires, for example, M input signals from M sensors 30 (M>0). Examples of the input signal include pH, dissolved oxygen amount, temperature, optical spectrum, RGB value, potential (electrode potential), agitator rotation speed (Agit), and the like, but are not limited thereto. The input unit 2 may acquire a signal stored in the database 40 as an input signal.

The control unit 6 determines from which sensor 30 the input signal data was acquired. For example, the control unit 6 may distinguish the sensor 30 by reading a sensor ID added to the input signal. The control unit 6 performs setting of the sensor 30 throughout a culture period so that the input signal becomes optimal. For example, the control unit 6 sets a range of the sensor 30 that outputs an optical spectrum according to a culture stage. Since transmittance of the culture medium is high in an early stage of culture, the control unit 6 narrows the range (shortens exposure time). Since the transmittance of the culture medium decreases as culture progresses, the control unit widens the range (lengthens exposure time).

The setting unit 7 sets an input criterion indicating performance required for data analysis. The setting unit 7 sets an index of the input signal corresponding to the evaluation index as the input criterion. The setting unit 7 may acquire the evaluation index from, for example, the database 40 or an external device, or may receive input of the evaluation index. In one example, the input criterion may be a signal-to-noise ratio (SNR).

The preprocessing unit 3 performs a preprocessing operation on the input signal for comparison with the input criterion to generate a preprocessed signal. That is, the preprocessing unit 3 processes the input signal to correspond to a format of the input criterion. For example, the preprocessing unit 3 may perform gain adjustment, calibration, and the like as the preprocessing operation. The preprocessing unit 3 may perform an operation using a plurality of input signals as the preprocessing operation. The preprocessing unit 3 may calculate SNR as the preprocessing operation.

The comparison unit 8 compares a signal with an input criterion corresponding to the signal to generate a comparison result. The comparison result may indicate whether or not the signal satisfies the input criterion. The comparison unit 8 may compare the preprocessed signal with an input criterion corresponding to the preprocessed signal to generate a comparison result. The comparison unit 8 may compare the postprocessed signal with an input criterion corresponding to the postprocessed signal to generate a comparison result.

The postprocessing unit 4 performs a postprocessing operation using another signal obtained by measuring the measurement object on the preprocessed signal when the preprocessed signal does not satisfy the input criterion, to generate a postprocessed signal. For example, the postprocessing operation may be acquisition of an arithmetic mean or moving average of signals. When the preprocessed signal satisfies the input criterion, the postprocessing unit 4 does not perform the postprocessing operation. That is, when the preprocessed signal satisfies the input criterion, the postprocessed signal is not generated.

The extraction unit 5 extracts extracted data based on the comparison result from the comparison unit 8. The extraction unit 5 extracts the preprocessed signal as the extracted data when the preprocessed signal satisfies the input criterion. The extraction unit 5 extracts the postprocessed signal as the extracted data when the postprocessed signal satisfies the input criterion. The extraction unit 5 may extract data obtained by performing an operation for output or the like on the preprocessed signal or the postprocessed signal as the extracted data. The extraction unit 5 may remove the postprocessed signal when the postprocessed signal does not satisfy the input criterion. That is, the extraction unit 5 performs conversion and selection of data.

The abnormality determination unit 9 determines abnormality based on the input signal, the preprocessed signal, or the postprocessed signal. For example, the abnormality determination unit 9 may determine abnormality based on a change in the input signal or correlation between input signals. In one example, the abnormality determination unit 9 may detect abnormality based on a change in dissolved oxygen amount. In another example, the abnormality determination unit 9 may detect abnormality based on correlation between the dissolved oxygen amount or pH and the electrode potential. The abnormality determination unit 9 may invalidate the extracted data as processing at the time of abnormality. "Invalidate the extracted data" may be to replace the preprocessed signal, the postprocessed signal, or the extracted data with None data.

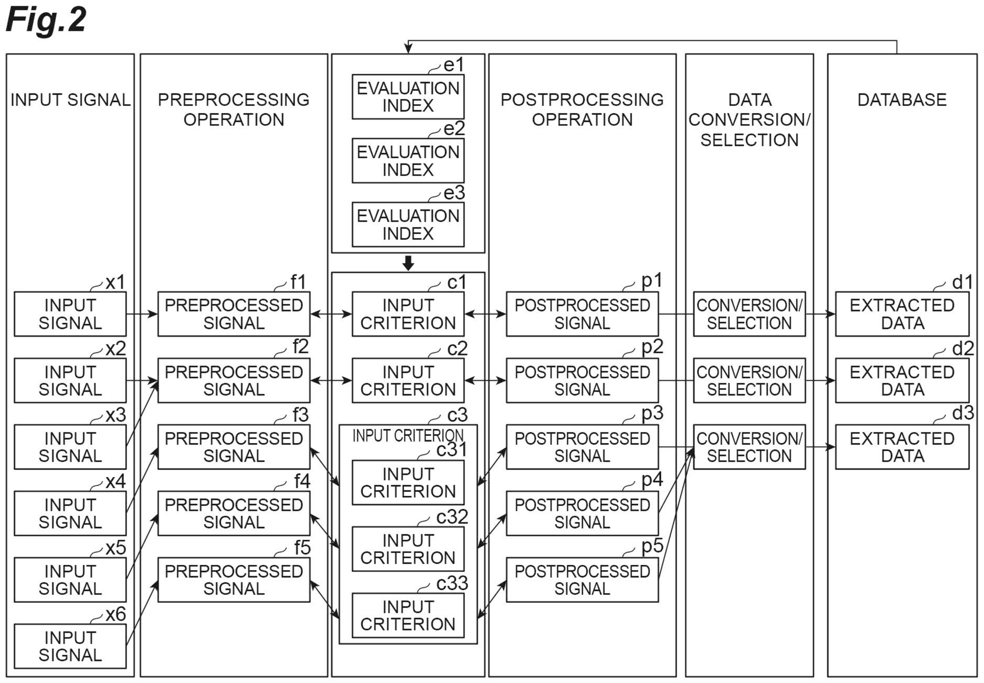

How the evaluation index and the input criterion are determined may differ depending on contents of the input signal. With reference to FIG. 2, examples of three cases (a first case, a second case, and a third case) of the evaluation index will be described. FIG. 2 is a schematic diagram showing an example of operation of the data extraction system 1. In the example shown in FIG. 2, three pieces of extracted data are extracted from six input signals.

In the first case, an evaluation index e1 for evaluating one type of signal is used. The setting unit 7 converts the evaluation index e1 into an input criterion c1. The evaluation index e1 may be an index similar to the input criterion c1.

In one example, when a certain aqueous solution changes in concentration by 50 mM (mmol/L), a SNR with respect to a rate of change in absorbance necessary to ascertain this change may be required as the evaluation index e1. When the evaluation index e1 is assumed to be 20 dB, the setting unit 7 calculates a SNR (input criterion c1) necessary for transmitted light by, for example, the following formula.

$$\Delta A \, (\text{SNR}) = 20 \text{ dB}$$

$$20 \times \text{LOG} \, (\Delta S/N) - 6 \, (\text{dB}) = 20 \text{ dB}$$

Here, $\Delta A$ is a difference in absorbance. $\Delta S$ is a difference in transmitted light intensity. N is base noise that an optical sensor has. 6 dB is a deterioration in SNR of transmitted light S before $\Delta A$ can be calculated. When $\Delta S = S \times 1\%$, the SNR necessary for transmitted light (S) is calculated as 66 dB (= input criterion c1). The setting unit 7 converts the evaluation index e1 into the input criterion c1 by performing such calculation on the evaluation index e1. The setting unit 7 may always calculate up to absorbance in preprocessing and make the evaluation index e1 and the input criterion c1 coincide. Such selection may be performed on a case-by-case basis depending on a type of culture and an element to be monitored.

In one example, there is a case of culture in which a change in absorbance is monitored based on absorbance at an early stage of culture, and the change is small in the early stage of culture and large from a middle stage to a later stage of culture. In this case, the setting unit 7 may separate an input criterion for transmitted light at an initial stage, which serves as a reference value, and an input criterion for transmitted light after a certain period of time has elapsed, even if an evaluation criterion for the change ∆A in absorbance is always the same. For example, the setting unit 7 may extract data with an input criterion having high SNR in the early stage of culture where the change is small, and may lower SNR for transmitted light from the middle stage of culture onward where the change is large. In another example, there is a case of monitoring the change (∆A) in absorbance at constant intervals at all times. In this case, the setting unit 7 may make the evaluation index e1 and the input criterion c1 coincide. As described above, by setting an input criterion corresponding to each case, optimal data extraction becomes possible.

The input unit 2 acquires an input signal x1. The input signal x1 is, for example, an optical spectrum. The preprocessing unit 3 performs a preprocessing operation such as gain adjustment and calibration on the input signal x1 to generate a preprocessed signal f1. For example, gain adjustment for an optical spectrum absorbs a difference in exposure time. Calibration for an optical spectrum is dark correction. In another example, at least one of gain adjustment and calibration may be performed corresponding to a type of the input signal x1 or a type of the sensor 30. The comparison unit 8 compares the preprocessed signal f1 with the input criterion c1 to generate a comparison result.

When the preprocessed signal f1 does not satisfy the input criterion c1, the postprocessing unit 4 performs a postprocessing operation such as arithmetic mean or moving average on the preprocessed signal f1 to generate a postprocessed signal p1. The comparison unit 8 compares the postprocessed signal p1 with the input criterion c1 to generate a comparison result.

When the preprocessed signal f1 satisfies the input criterion c1, the extraction unit 5 extracts the preprocessed signal f1 as extracted data d1. When the postprocessed signal p1 satisfies the input criterion c1, the extraction unit 5 extracts the postprocessed signal p1 as the extracted data d1. When the postprocessed signal p1 does not satisfy the input criterion c1, the extraction unit 5 removes the postprocessed signal p1. For example, the extraction unit 5 may remove the postprocessed signal pl by replacing the postprocessed signal p1 with None data. The extraction unit 5 may extract None data as the extracted data d1. The database 40 stores the extracted data d1.

In the second case, an evaluation index e2 for evaluating a result of operating a plurality of types of signals is used. The setting unit 7 converts the evaluation index e2 into an input criterion c2.

The input unit 2 acquires input signals x2 and x3. The input signal x2 is, for example, a natural potential of a platinum electrode with respect to GND. GND is a potential serving as a reference of a measurement system. The natural potential is a potential generated by a metal electrode immersed in an aqueous solution due to an equilibrium state of oxidation-reduction. The input signal x3 is, for example, a reference electrode potential that is also used in pH measurement. This is obtained by immersing silver/silver chloride (an electrode obtained by oxidizing a surface of silver with silver chloride) in KCL (potassium chloride solution), and by making concentrations of Ag ions and Cl ions constant, a stable equilibrium potential "Ag(+) + Cl(-) = AgCl" is obtained, and is a potential serving as a reference for potential measurement.

When a platinum electrode potential (E) with respect to the reference electrode is represented as ∆E as to how it has changed from an early stage of culture, a SNR with respect to a rate of change in potential necessary to ascertain a change in culture solution may be required as the evaluation index e2. When the evaluation index e2 is assumed to be 20 dB, the setting unit 7 calculates SNR (input criterion c2) related to potential by, for example, the following formula.

$$\Delta E \text{ (SNR)} = 20 \text{ dB}$$

$$20 \times \text{LOG} (\Delta E/N) - 3 \text{ (dB)} = 20 \text{ dB}$$

Here, ∆E is a difference in potential. N is base noise that a potential sensor has. 3 dB is SNR deterioration due to a difference operation with the reference electrode. When ∆E = E x 10%, the SNR necessary for transmitted light (S) is calculated as 43 dB (= c1). The setting unit 7 converts the evaluation index e2 into the input criterion c2 by performing such calculation on the evaluation index e2.

The preprocessing unit 3 subtracts the input signal x3 from the input signal x2 to generate a preprocessed signal f2 that is a potential of the input signal x2 (platinum electrode) with respect to a reference potential of the input signal x3. By arranging a large number of certain metal electrodes, the input signal x2 and the extracted data d2 may each be converted into multisignals. The comparison unit 8 compares the preprocessed signal f2 with the input criterion c2 to generate a comparison result.

When the preprocessed signal f2 does not satisfy the input criterion c2, the postprocessing unit 4 performs a postprocessing operation such as arithmetic mean or moving average on the preprocessed signal f2 to generate a postprocessed signal p2. The comparison unit 8 compares the postprocessed signal p2 with the input criterion c2 to generate a comparison result.

When the preprocessed signal f2 satisfies the input criterion c2, the extraction unit 5 extracts the preprocessed signal f2 as extracted data d2. When the postprocessed signal p2 satisfies the input criterion c2, the extraction unit 5 extracts the postprocessed signal p2 as the extracted data d2. When the postprocessed signal p2 does not satisfy the input criterion c2, the extraction unit 5 removes the postprocessed signal p2. For example, the extraction unit 5 may remove the postprocessed signal p2 by replacing the postprocessed signal p2 with None data. The extraction unit 5 may extract None data as the extracted data d2. The database 40 stores the extracted data d2.

In the third case, an evaluation index e3 for evaluating a plurality of types of signals respectively is used. The input unit 2 acquires input signals x4 to x6. The input signals x4 to x6 are each RGB signals (for example, luminance values of R, G, and B in an RGB color model). When RGB signals are input, a final signal may be extracted as a value of $\Delta E^*ab$ in an $L^*a^*b^*$ color space. In this case, the SNR necessary for the value of $\Delta E^*ab$ is set as the evaluation index e3. The setting unit 7 sets SNR necessary for each of the RGB signals as input criteria c31 to c33 (input criterion c3).

The preprocessing unit 3 performs a preprocessing operation such as gain adjustment and white balance adjustment on each of the input signals x4 to x6 to generate preprocessed signals f3 to f5. For example, white balance adjustment for luminance values of R, G, and B is calibration for individual variation. The preprocessed signal f3 corresponds to the input criterion c31. The preprocessed signal f4 corresponds to the input criterion c32. The preprocessed signal f5 corresponds to the input criterion c33. The comparison unit 8 compares the preprocessed signal f3 with the input criterion c31, compares the preprocessed signal f4 with the input criterion c32, and compares the preprocessed signal f5 with the input criterion c33 to generate comparison results.

When at least one of the preprocessed signals f3 to f5 does not satisfy the input criteria c31 to c33 respectively corresponding thereto, the postprocessing unit 4 performs a postprocessing operation such as arithmetic mean or moving average on the preprocessed signals f3 to f5 to generate postprocessed signals p3 to p5. The postprocessed signal p1 corresponds to the input criterion c31. The postprocessed signal p2 corresponds to the input criterion c32. The postprocessed signal p3 corresponds to the input criterion c33. The comparison unit 8 compares the postprocessed signal p3 with the input criterion c31, compares the postprocessed signal p4 with the input criterion c32, and compares the postprocessed signal p5 with the input criterion c33 to generate comparison results.

When the preprocessed signals f3 to f5 satisfy the input criteria c31 to c33 respectively corresponding thereto, the extraction unit 5 extracts the preprocessed signals f3 to f5 as extracted data d3. When the postprocessed signals p3 to p5 satisfy the input criteria c31 to c33 respectively corresponding thereto, the extraction unit 5 extracts the postprocessed signals p3 to p5 as the extracted data d3. The extraction unit 5 may extract data obtained by performing an operation for output or the like on the preprocessed signals f3 to f5 or the postprocessed signals p3 to p5 as the extracted data d3. The extraction unit 5 may calculate a value of $\Delta E^*ab$ in an $L^*a^*b^*$ color space using the preprocessed signals f3 to f5 or the postprocessed signals p3 to p5, and extract the value as the extracted data d3. When at least one of the postprocessed signals p3 to p5 does not satisfy the input criteria c31 to c33 respectively corresponding thereto, the extraction unit 5 removes the postprocessed signals p3 to p5. For example, the extraction unit 5 may remove the postprocessed signals p3 to p5 by replacing the postprocessed signals p3 to p5 with None data. The extraction unit 5 may extract None data as the extracted data d3. The database 40 stores the extracted data d3.

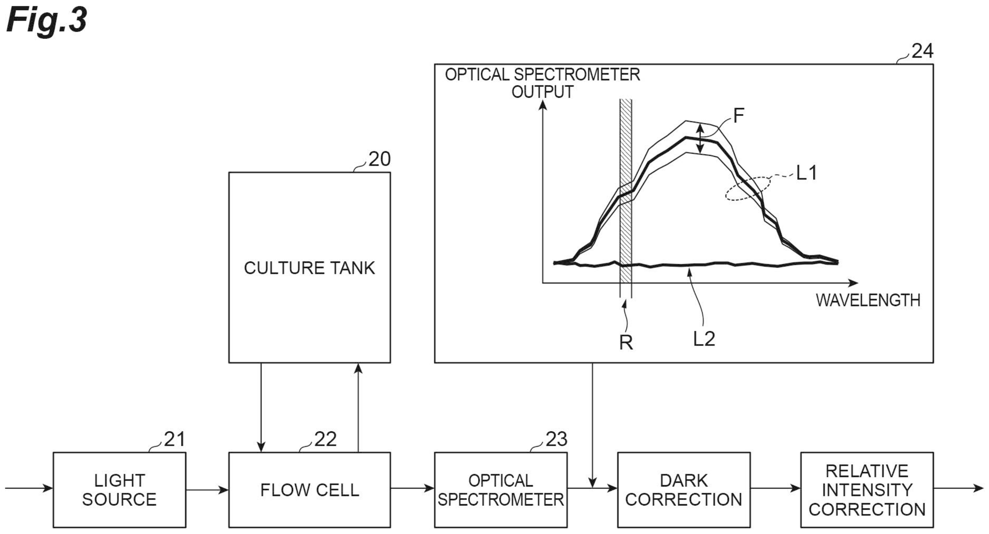

FIG. 3 is a diagram showing an example of optical spectroscopic measurement in bioproduction. In the example shown in FIG. 3, optical spectroscopic measurement is performed using a culture tank 20, a light source 21, a flow cell 22, and an optical spectrometer 23. The light source 21 irradiates the flow cell 22 with light. The flow cell 22 draws out and circulates a culture medium from the culture tank 20. Transmitted light from the flow cell 22 is incident on the optical spectrometer 23. The optical spectrometer 23 outputs an optical spectrum.

A graph 24 shown in FIG. 3 schematically shows an output of the optical spectrometer 23. The graph 24 shows a signal level group L1 when optical spectroscopic measurement is performed a plurality of times, and a signal level L2 at the time of dark. Even if a state of the culture medium flowing through the flow cell 22 is constant, a signal level (signal output) fluctuates. As a result, a fluctuation width F may exist in the signal level group L1. This fluctuation width F is caused by, for example, installation conditions of hardware such as an optical fiber related to optical spectroscopic measurement. In the example shown in FIG. 3, dark correction and relative intensity correction are performed as the preprocessing operation.

The preprocessing unit 3 performs dark correction as a preprocessing operation on the optical spectrum. For example, the preprocessing unit 3 subtracts the signal level L2 at the time of dark from the signal level of the optical spectrum. Thereby, a signal level that changes depending on a state of the culture medium flowing through the flow cell 22 is derived.

The preprocessing unit 3 may further perform relative intensity correction as a preprocessing operation on the signal on which dark correction has been performed. For example, the preprocessing unit 3 calculates relative intensity of the whole using a signal in a wavelength range R that does not respond to a change in the culture medium and where a transmittance does not become zero (signal level is not zero). "Does not respond to a change in the culture medium" means that the signal level does not fluctuate even if there is a change in the culture medium. For example, until light passes through an object, a manner of absorption of each wavelength of light is different. This is because absorption wavelength differs depending on a molecular structure of the object. At a wavelength that does not react to any molecular structure, light "does not respond to a change in the culture medium", that is, becomes "a wavelength at which the signal level does not fluctuate even if there is a change in the culture medium". As the wavelength range R that does not respond to a change in the culture medium and where a transmittance does not become zero, for example, a wavelength range around 1300 nm can be cited. The preprocessing unit 3 calculates relative intensity with respect to the signal level in the wavelength range R using the signal on which dark correction has been performed. That is, the preprocessing unit 3 generates the preprocessed signal by calculating a relative intensity with the signal level in a wavelength range that does not respond to a change in the culture medium and where a transmittance does not become zero.

The optical spectroscopic measurement may be near-infrared spectroscopic measurement. At the time of near-infrared spectroscopic measurement, there exists a wavelength range (for example, 1250 to 1350 nm) that does not respond to organic molecule fluctuation and where water absorption is not too large. "Does not respond to organic molecule fluctuation" means that the signal level does not fluctuate even if there is fluctuation of organic molecules, or the signal level is not affected by the fluctuation of organic molecules. "Water absorption is not too large" means that light is transmitted with a certain degree of intensity and light intensity can be stably observed by the optical spectrometer 23. In other words, it means that light intensity (signal level) necessary for near-infrared spectroscopic measurement is not affected by water absorption. Here, a range of appropriate absorbance depends on a measurement target, but is, for example, 0.05 to 1.5. A wavelength range where water absorption is not too large may be, for example, a range where absorbance is 1.0 (absorption rate is 90%) or less. A wavelength range that does not respond to organic molecule fluctuation and where water absorption is not too large is an example of the wavelength range R. The preprocessing unit 3 calculates relative intensity with respect to the signal level in the wavelength range using the signal on which dark correction has been performed. That is, the preprocessing unit 3 generates the preprocessed signal by calculating a relative intensity with the signal level in a wavelength range that does not respond to organic molecule fluctuation and where water absorption is not too large as the wavelength range R.

In the example shown in FIG. 3, the input unit 2 and the preprocessing unit 3 function as a partial data extraction system or sensing system. The data extraction system acquires an optical spectrum measured by optical spectroscopic measurement in bioproduction. The data extraction system outputs a signal obtained by calculating a relative intensity of the optical spectrum relative to the signal level in a wavelength range that does not respond to a change in the culture medium and where a transmittance does not become zero.

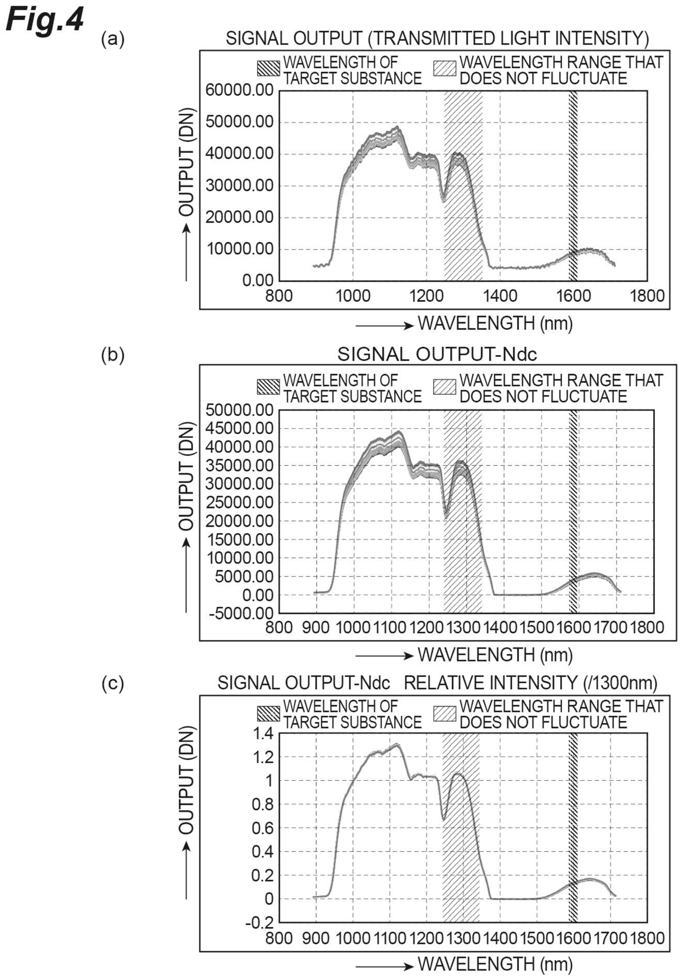

Relative intensity correction will be described with reference to FIG. 4(a) to (c). FIG. 4(a) to (c) are graphs exemplifying extraction of a change in organic molecule concentration in near-infrared spectroscopic measurement. In FIG. 4(a) to (c), a vertical axis indicates a signal level, and a horizontal axis indicates a wavelength. FIG. 4(a) to (c) show a wavelength range (for example, 1250 to 1350 nm) that does not respond to organic molecule fluctuation and where water absorption is not too large as "a wavelength range that does not fluctuate". Moreover, FIG. 4(a) to (c) show a calibration curve (1600 nm) of glucose as "a wavelength of a target substance".

FIG. 4(a) is a graph showing a signal level obtained by circulating tap water in the flow cell 22 instead of the culture medium and performing near-infrared spectroscopic measurement for several hours. FIG. 4(a) shows that the signal level of near-infrared spectroscopy fluctuates.

FIG. 4(b) is a graph showing a signal level on which dark correction has been performed on the signal level shown in FIG. 4(a). That is, FIG. 4(b) is an example in which the signal level at the time of dark has been subtracted from the signal level shown in FIG. 4(a). When comparing FIG. 4(b) with FIG. 4(a), it can be said that offset is eliminated by dark correction.

FIG. 4(c) is a graph showing a signal level converted into relative intensity using the signal level around 1300 nm (for example, 1250 to 1350 nm) shown in FIG. 4(b). Each of the signal levels shown in FIG. 4(c) exhibits a similar shape. That is, it can be said that fluctuation in signal level is eliminated by relative intensity correction. Therefore, it becomes easy to capture a change in the wavelength range of the target substance by the signal level converted into relative intensity.

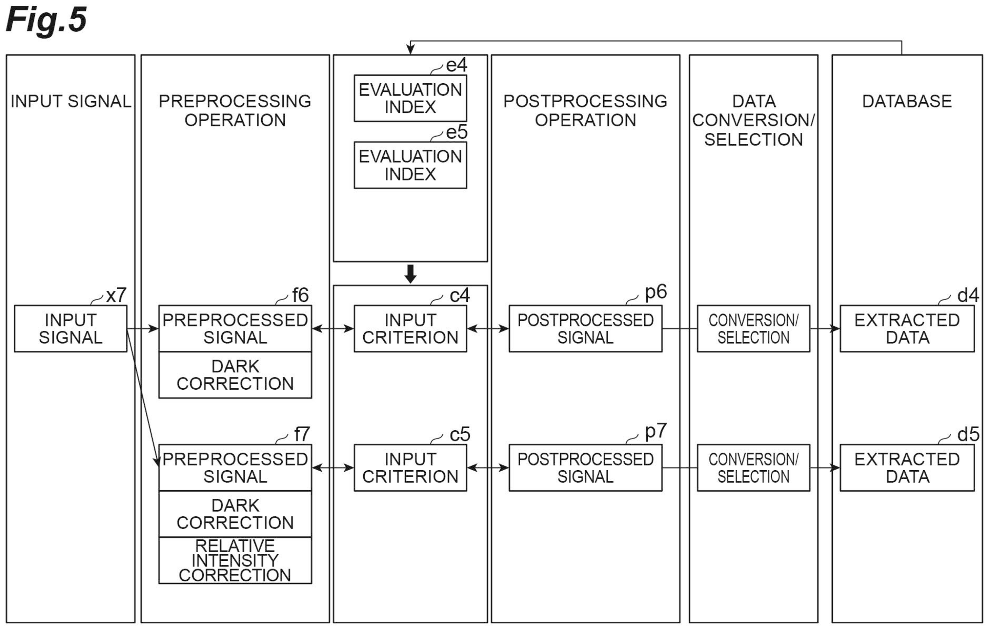

FIG. 5 is a schematic diagram showing another example of operation of the data extraction system. FIG. 5 shows an example of acquiring an input signal using the configuration shown in FIG. 3. In FIG. 5, evaluation indexes e4 and e5 for evaluating SNR are used. The setting unit 7 sets an input criterion c4 corresponding to the evaluation index e4. The setting unit 7 sets an input criterion c5 corresponding to the evaluation index e5. The input unit 2 acquires an input signal x7 from the optical spectrometer 23. The input signal x7 is an optical spectrum. The setting unit 7 converts the evaluation indexes e4 and e5 into the input criteria c4 and c5 in the same manner as a calculation method of the input criterion c1 based on the evaluation index e1.

In one example, a SNR with respect to a rate of change in absorbance necessary to ascertain a change in turbidity of a certain aqueous solution may be required as the evaluation index e4. When the evaluation index e4 is assumed to be 10 dB, the setting unit 7 calculates a SNR (input criterion c4) necessary for transmitted light by, for example, the following formula.

$$\Delta A\,(SNR) = 10\;dB$$

$$20 \times LOG\,(\Delta S/N) - 6\,(dB) = 10\;dB$$

Here, ΔA is a difference in absorbance. ΔS is a difference in transmitted light intensity. N is base noise that an optical sensor has. 6 dB is a deterioration in SNR of transmitted light S before ΔA can be calculated. When ΔS = S x 4%, a SNR necessary for transmitted light (S) is calculated as 44 dB (= input criterion c4). The setting unit 7 converts the evaluation index e4 into the input criterion c4 by performing such calculation on the evaluation index e4. The setting unit 7 converts the evaluation index e5 into the input criterion c5 by performing similar calculation on the evaluation index e5 with, for example, ΔA (SNR) = 20 dB.

The preprocessing unit 3 performs dark correction on the input signal x7 to generate a preprocessed signal f6. When the preprocessed signal f6 does not satisfy the input criterion c4, the postprocessing unit 4 performs a postprocessing operation on the preprocessed signal f6 to generate a postprocessed signal p6. The input signal x7 on which dark correction has been performed is finally extracted as extracted data d4.

The preprocessing unit 3 performs dark correction and relative intensity correction on the input signal x7 to generate a preprocessed signal f7. When the preprocessed signal f7 does not satisfy the input criterion c5, the postprocessing unit 4 performs a postprocessing operation on the preprocessed signal f7 to generate a postprocessed signal p7. The input signal x7 on which dark correction and relative intensity correction have been performed is finally extracted as extracted data d5.

As in the example shown in FIG. 5, the extracted data d4 and d5 may be extracted by branching processing based on the input signal x7. The extracted data d4 and d5 can be used according to a purpose of data analysis when substances to be grasped as a change in the culture medium are different.

It can be said that the example shown in FIG. 5 is processing for extracting data obtained by performing different preprocessing operations on the same signal. The preprocessing unit 3 generates a first preprocessed signal (preprocessed signal f6) for which relative intensity is not calculated, and generates a second preprocessed signal (preprocessed signal f7) for which relative intensity is calculated. The setting unit 7 sets a first input criterion (input criterion c4) that is an input criterion corresponding to the first preprocessed signal, and sets a second input criterion (input criterion c5) that is an input criterion corresponding to the second preprocessed signal. The postprocessing unit 4 performs a postprocessing operation on the first preprocessed signal when the first preprocessed signal does not satisfy the first input criterion, to generate a first postprocessed signal (postprocessed signal p6). The extraction unit 5 extracts the first preprocessed signal as first extracted data (extracted data d4) when the first preprocessed signal satisfies the first input criterion, and extracts the first postprocessed signal as the first extracted data when the first postprocessed signal satisfies the first input criterion. The postprocessing unit 4 performs a postprocessing operation on the second preprocessed signal when the second preprocessed signal does not satisfy the second input criterion, to generate a second postprocessed signal (postprocessed signal p7). The extraction unit 5 extracts the second preprocessed signal as second extracted data (extracted data d5) when the second preprocessed signal satisfies the second input criterion, and extracts the second postprocessed signal as the second extracted data when the second postprocessed signal satisfies the second input criterion.

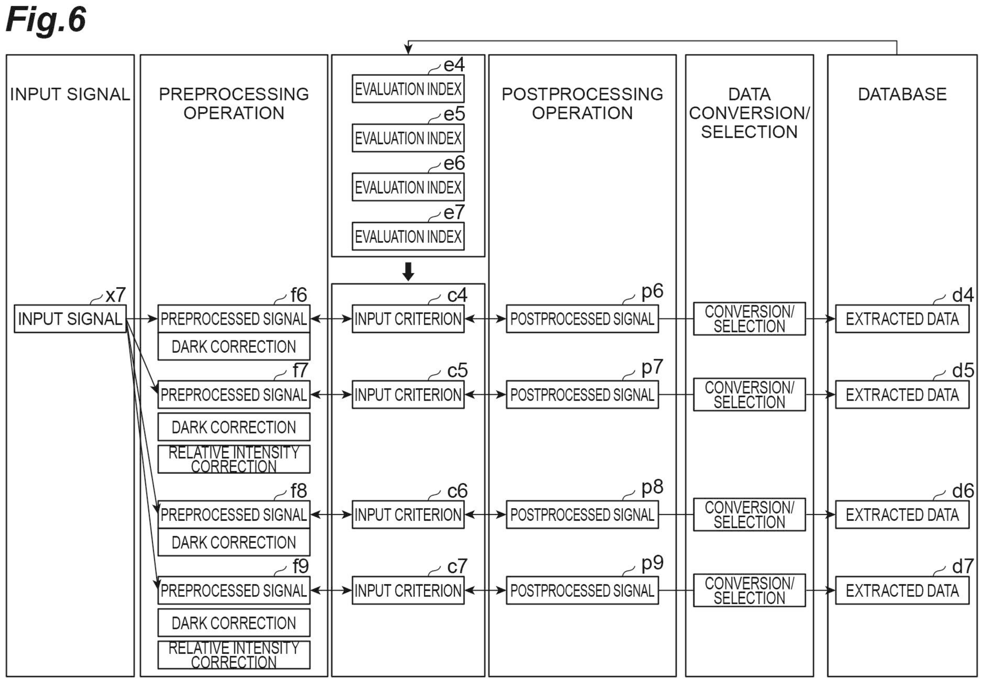

FIG. 6 is a schematic diagram showing still another example of operation of the data extraction system. FIG. 6 shows an example in which different input criteria are set in addition to the processing shown in FIG. 5. In FIG. 6, evaluation indexes e4 to e7 for evaluating SNR are used. The setting unit 7 sets an input criterion c4 corresponding to the evaluation index e4. The setting unit 7 sets an input criterion c5 corresponding to the evaluation index e5. The setting unit 7 sets an input criterion c6 corresponding to the evaluation index e6. The setting unit 7 sets an input criterion c7 corresponding to the evaluation index e7. The input unit 2 acquires an input signal x7 from the optical spectrometer 23. The input signal x7 is an optical spectrum.

For example, the setting unit 7 calculates a SNR (input criteria c4 to c7) necessary for transmitted light by, for example, the following formulas.

- In the case of input criterion c4

$$\Delta A\,(SNR) = 10\;dB$$

$$20 \times LOG\,(\Delta S/N) - 6\,(dB) = 10\;dB$$

- In the case of input criterion c5

$$\Delta A\,(SNR) = 20\;dB$$

$$20 \times LOG\,(\Delta S/N) - 6\,(dB) = 20\;dB$$

- In the case of input criterion c6

$$\Delta A\,(SNR) = 0\;dB$$

$$20 \times LOG\,(\Delta S/N) - 6\,(dB) = 0\;dB$$

- In the case of input criterion c7

$$\Delta A \; (SNR) = 10 \; dB$$

$$20 \times LOG \; (\Delta S/N) - 6 \; (dB) = 10 \; dB$$

Here, $\Delta A$ is a difference in absorbance. $\Delta S$ is a difference in transmitted light intensity. N is base noise that an optical sensor has. 6 dB is a deterioration in SNR of transmitted light S before $\Delta A$ can be calculated. In the case of input criterion c4, when $\Delta S = S \times 4\%$, a SNR necessary for transmitted light (S) is calculated as 44 dB. In the case of input criterion c5, when $\Delta S = S \times 1\%$, a SNR necessary for transmitted light (S) is calculated as 66 dB. In the case of input criterion c6, when $\Delta S = S \times 4\%$, a SNR necessary for transmitted light (S) is calculated as 34 dB. In the case of input criterion c7, when $\Delta S = S \times 1\%$, a SNR necessary for transmitted light (S) is calculated as 54 dB. The setting unit 7 converts the evaluation indexes e4 to e7 into the input criteria c4 to c7 by performing such calculation on the evaluation indexes e4 to e7.

The preprocessing unit 3 performs dark correction on the input signal x7 to generate a preprocessed signal f6. When the preprocessed signal f6 does not satisfy the input criterion c4, the postprocessing unit 4 performs a postprocessing operation on the preprocessed signal f6 to generate a postprocessed signal p6. The input signal x7 on which dark correction has been performed is finally extracted as extracted data d4.

The preprocessing unit 3 performs dark correction and relative intensity correction on the input signal x7 to generate a preprocessed signal f7. When the preprocessed signal f7 does not satisfy the input criterion c5, the postprocessing unit 4 performs a postprocessing operation on the preprocessed signal f7 to generate a postprocessed signal p7. The input signal x7 on which dark correction and relative intensity correction have been performed is finally extracted as extracted data d5.

The preprocessing unit 3 performs dark correction on the input signal x7 to generate a preprocessed signal f8. The preprocessed signal f8 is identical to the preprocessed signal f6. When the preprocessed signal f8 does not satisfy the input criterion c6, the postprocessing unit 4 performs a postprocessing operation on the preprocessed signal f8 to generate a postprocessed signal p8. The input signal x7 on which dark correction has been performed is finally extracted as extracted data d6.

The preprocessing unit 3 performs dark correction and relative intensity correction on the input signal x7 to generate a preprocessed signal f9. The preprocessed signal f9 is identical to the preprocessed signal f7. When the preprocessed signal f9 does not satisfy the input criterion c7, the postprocessing unit 4 performs a postprocessing operation on the preprocessed signal f9 to generate a postprocessed signal p9. The input signal x7 on which dark correction and relative intensity correction have been performed is finally extracted as extracted data d7.

The input criteria c4 and c6 are common in that both are used for comparison with a preprocessed signal on which dark correction has been performed, but their values are different from each other. That is, it can be said that the input criteria c4 and c6 are input criteria corresponding to the preprocessed signal on which dark correction has been performed, and are different input criteria from each other. When the SNR of the input criterion c4 is greater than the SNR of the input criterion c6, the SNR of the extracted data d4 becomes a high-precision signal higher than the SNR of the extracted data d6. Response of the extracted data d6 is higher than response of the extracted data d4. The response is determined based on time related to the postprocessing operation.

The input criteria c5 and c7 are common in that both are used for comparison with a preprocessed signal on which dark correction and relative intensity correction have been performed, but their values are different from each other. That is, it can be said that the input criteria c5 and c7 are input criteria corresponding to the preprocessed signal on which dark correction and relative intensity correction have been performed, and are different input criteria from each other. When the SNR of the input criterion c5 is greater than the SNR of the input criterion c7, the SNR of the extracted data d5 becomes a high-precision signal higher than SNR of the extracted data d7. Response of the extracted data d7 is higher than response of the extracted data d5.

It can be said that the example shown in FIG. 6 is processing for extracting data having different properties by setting different input criteria for the same signal. The setting unit 7 further sets another input criterion corresponding to the preprocessed signal. The postprocessing unit 4 performs a postprocessing operation on the preprocessed signal when the preprocessed signal does not satisfy the other input criterion, to further generate another postprocessed signal. The extraction unit 5 further extracts the preprocessed signal as other extracted data when the preprocessed signal satisfies the other input criterion, and further extracts the postprocessed signal as other extracted data when the other postprocessed signal satisfies the other input criterion.

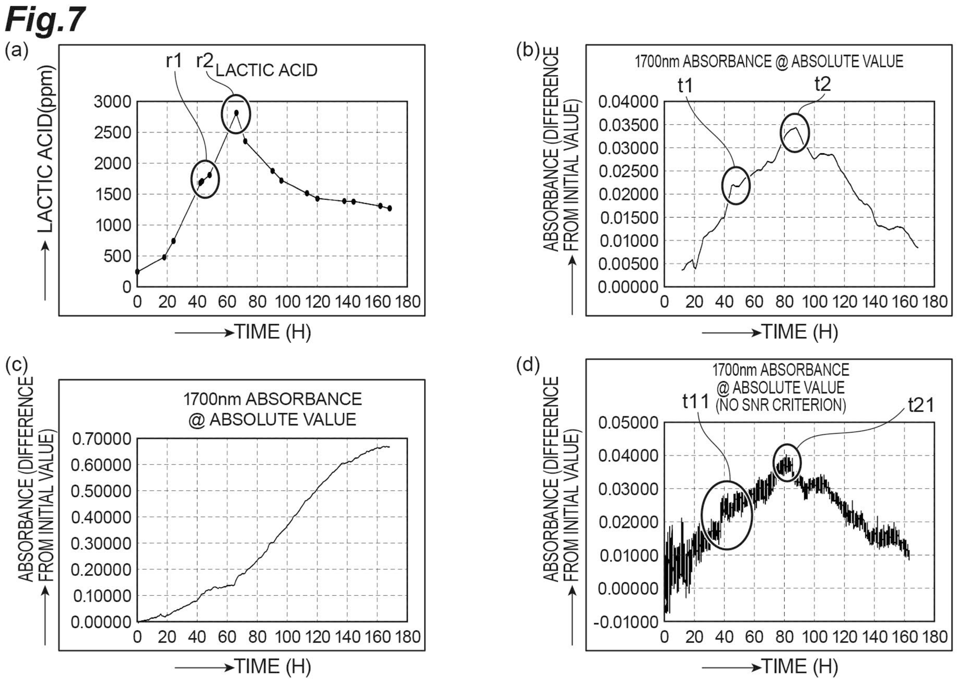

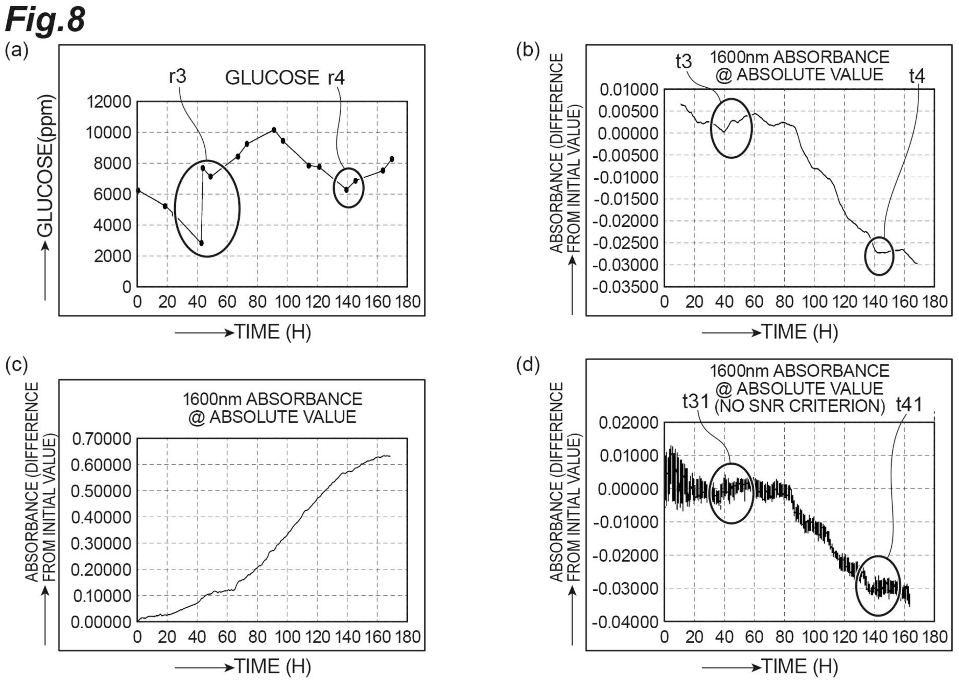

With reference to FIG. 7(a) to (d) and FIG. 8(a) to (d), an example of a result of improvement in quality of data used for analysis will be described as an effect of the present disclosure. FIG. 7(a) to (d) are graphs showing an example for explaining an effect by the preprocessing operation and setting of the input criterion. FIG. 7(a) to(d) show an example in which a culture experiment was performed for several days and lactic acid was observed as a target substance.

FIG. 7(a) shows an example in which concentration of lactic acid was measured by sampling by high performance liquid chromatography (HPLC: High Performance Liquid Chromatography). In FIG. 7(a), a vertical axis indicates concentration of lactic acid, and a horizontal axis indicates time. In FIG. 7(a), inflection points r1 and r2 appear.

FIG. 7(b) to (d) show examples in which data was extracted under different conditions based on data observed in real time every minute by near-infrared spectroscopy. In FIG. 7(b) to (d), a vertical axis indicates absorbance (difference from an initial value), and a horizontal axis indicates time.

FIG. 7(b) shows an example in which a value of 1700 nm (calibration curve of lactic acid) is extracted from extracted data on which SNR is applied as an input criterion and dark correction and relative intensity correction are performed as a preprocessing operation. When comparing FIG. 7(a) and (b), it can be seen that an inflection point t1 corresponding to the inflection point r1 and an inflection point t2 corresponding to the inflection point r2 are captured.

FIG. 7(c) shows an example in which a value of 1700 nm (calibration curve of lactic acid) is extracted from extracted data on which SNR is applied as an input criterion and dark correction is performed as a preprocessing operation. In FIG. 7(c), a signal monotonically increases, and a change in concentration of lactic acid is not known. However, the monotonic increase captures turbidity. Such monotonic increase in turbidity has an extremely high correlation with production amount of product generated by culture.

FIG. 7(d) shows an example in which a value of 1700 nm (calibration curve of lactic acid) is extracted from extracted data on which no input criterion is applied and dark correction and relative intensity correction are performed as a preprocessing operation. In FIG. 7(d), inflection points t11 and t21 are buried in noise. Such a signal can be said to be a noisy but highly sensitive signal. In a case of culture in which a large change occurs instantaneously, it is also useful to use a highly sensitive signal in data analysis.

FIG. 8(a) to (d) are graphs showing another example for explaining an effect by the preprocessing operation and setting of the input criterion. FIG. 8(a) to (d) show an example in which a culture experiment was performed for several days and glucose was observed as a target substance.

FIG. 8(a) shows an example in which concentration of glucose was measured by sampling by HPLC. In FIG. 8(a), a vertical axis indicates concentration of glucose, and a horizontal axis indicates time. In FIG. 8(a), inflection points r3 and r4 appear.

FIG. 8(b) to (d) show examples in which data was extracted under different conditions based on data observed in real time every minute by near-infrared spectroscopy. In FIG. 8(b) to (d), a vertical axis indicates absorbance (difference from an initial value), and a horizontal axis indicates time.

FIG. 8(b) shows an example in which a value of 1600 nm (calibration curve of glucose) is extracted from extracted data on which SNR is applied as an input criterion and dark correction and relative intensity correction are performed as a preprocessing operation. When comparing FIG. 8(a) and (b), it can be seen that an inflection point t3 corresponding to the inflection point r3 and an inflection point t4 corresponding to the inflection point r4 are captured.

FIG. 8(c) shows an example in which a value of 1600 nm (calibration curve of glucose) is extracted from extracted data on which SNR is applied as an input criterion and dark correction is performed as a preprocessing operation. In FIG. 8(c), a signal monotonically increases, and a change in concentration of glucose is not known. However, the monotonic increase captures turbidity. Such monotonic increase in turbidity has an extremely high correlation with production amount of product generated by culture.

FIG. 8(d) shows an example in which a value of 1600 nm (calibration curve of glucose) is extracted from extracted data on which no input criterion is applied and dark correction and relative intensity correction are performed as a preprocessing operation. In FIG. 8(d), inflection points t31 and t41 are buried in noise. Such a signal can be said to be a noisy but highly sensitive signal. In a case of culture in which a large change occurs instantaneously, it is also useful to use a highly sensitive signal in data analysis.

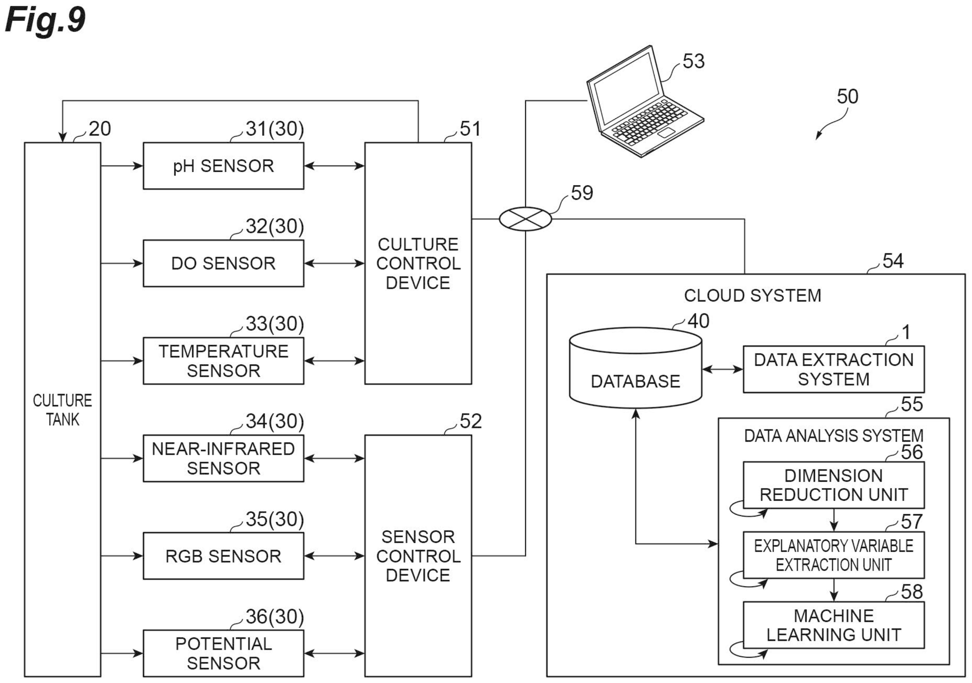

[Example] FIG. 9 is an overall diagram showing an example of application of the data extraction system 1 according to an example. A bioproduction system 50 shown in FIG. 9 includes a culture tank 20, a plurality of sensors 30 (a pH sensor 31, a DO sensor 32, a temperature sensor 33, a near-infrared sensor 34, an RGB sensor 35, and a potential sensor 36), a culture control device 51, a sensor control device 52, a control terminal 53, and a cloud system 54. The control terminal 53 is communicably connected to the culture control device 51 and the sensor control device 52 via a communication network 59. The cloud system 54 is communicably connected to the culture control device 51 and the sensor control device 52 via the communication network 59. The communication network 59 may be configured by either wired or wireless. The communication network 59 may be a non-dedicated line such as an Internet line and a mobile communication network, or may be a dedicated line.

The pH sensor 31, the DO sensor 32, and the temperature sensor 33 acquire data indispensable for culture (hereinafter referred to as "culture data"). The culture data may include agitator rotation speed (Agit) acquired by a torque sensor or the like. The culture control device 51 controls the culture tank 20, the pH sensor 31, the DO sensor 32, and the temperature sensor 33. The culture control device 51 adds sensor data information or the like to signals output from the pH sensor 31, the DO sensor 32, and the temperature sensor 33 and transmits them to the cloud system 54. Examples of the sensor data information include, but are not limited to, a version number and a serial number.

The near-infrared sensor 34, the RGB sensor 35, and the potential sensor 36 are used for making bioproduction AI (Artificial Intelligence). The making of bioproduction into AI includes, for example, predicting an optimal control value in real time using a machine learning model, and automatically controlling culture according to the prediction. The sensor control device 52 controls settings of the near-infrared sensor 34, the RGB sensor 35, and the potential sensor 36. The sensor control device 52 adds sensor data information or the like to signals output from the near-infrared sensor 34, the RGB sensor 35, and the potential sensor 36 and transmits them to the cloud system 54.

The control terminal 53 is one or more computers used by a user of the bioproduction system 50. The control terminal 53 is, for example, a personal computer, but is not limited thereto. The control terminal 53 receives various settings and various controls based on, for example, Web UI (User Interface). The control terminal 53 changes settings of the culture control device 51, the sensor control device 52, and the cloud system 54. The control terminal 53 controls the culture control device 51 and the sensor control device 52.

The cloud system 54 includes the data extraction system 1, the database 40, and a data analysis system 55. The database 40 may store signals received from the culture control device 51 and the sensor control device 52 as data. The data extraction system 1 may acquire a signal read from the database 40 as an input signal, process the signal, and store extracted data in the database 40.

The extracted data includes a plurality of pieces of data for each sensor 30. Furthermore, the extracted data is extracted as a plurality of pieces of data depending on patterns of the preprocessing operation, the input criterion, and the postprocessing operation, and parameters of a very large number of dimensions exist. The data analysis system 55 processes the extracted data read from the database 40 to perform data analysis. The data analysis system 55 includes, as functional elements, a dimension reduction unit 56, an explanatory variable extraction unit 57, and a machine learning unit 58.

The dimension reduction unit 56 compresses dimensions of the extracted data by, for example, principal component analysis or the like. The explanatory variable extraction unit 57 determines an explanatory variable from a dimension-compressed signal based on correlation with an objective variable in machine learning, regression analysis, or the like. The machine learning unit 58 performs machine learning using the determined explanatory variable to create a machine learning model. The bioproduction system 50 automatically controls culture in real time using the machine learning model.

The extracted data of the present disclosure is data that satisfies the input criterion, and therefore quality of the data is ensured. By using such extracted data for machine learning, efficient machine learning can be realized.

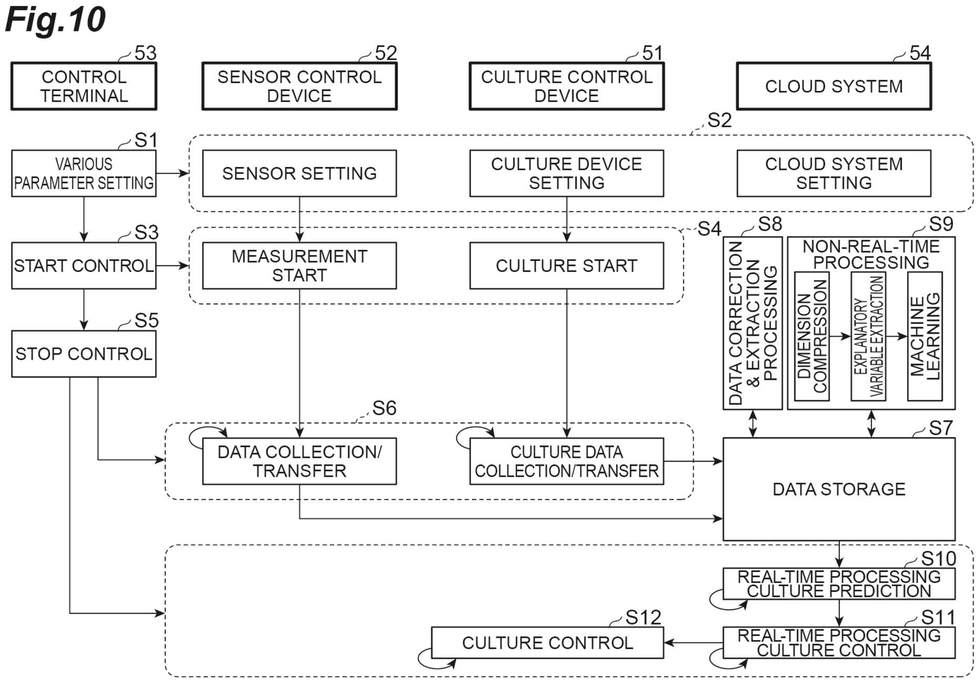

An example of operation of the bioproduction system 50 will be described with reference to FIG. 10. FIG. 10 is a diagram showing an example of overall operation of the bioproduction system 50 including the data extraction system 1.

In step S1, the control terminal 53 sets parameters of the culture control device 51, the sensor control device 52, and the cloud system 54. Examples of the parameters include, but are not limited to, culture type, culture time, respective set values of sensors (sampling interval, integration time, and gain), and ID for specifying culture data.

In step S2, the culture control device 51, the sensor control device 52, and the cloud system 54 each perform various settings in response to parameter setting from the control terminal 53.

In step S3, the control terminal 53 performs start control for starting operation of the culture control device 51 and the sensor control device 52. For example, the control terminal 53 may perform the start control by transmitting a command instructing start of operation to the culture control device 51 and the sensor control device 52 via Web UI.

In step S4, the culture control device 51 and the sensor control device 52 start operation in response to the start control from the control terminal 53, respectively. The culture control device 51 controls the pH sensor 31, the DO sensor 32, and the temperature sensor 33 to start culture. The sensor control device 52 controls the near-infrared sensor 34, the RGB sensor 35, and the potential sensor 36 to start measurement of the culture tank 20.

In step S5, the control terminal 53 performs stop control for stopping operation of the culture control device 51 and the sensor control device 52. For example, the control terminal 53 may perform the stop control by transmitting a command instructing stop of operation to the culture control device 51 and the sensor control device 52 via Web UI.

In step S6, the culture control device 51 and the sensor control device 52 transfer collected data to the cloud system 54 in response to the start control and the stop control from the control terminal 53, respectively. For example, the culture control device 51 adds sensor data information or the like to signals output from the pH sensor 31, the DO sensor 32, and the temperature sensor 33 and transmits them to the cloud system 54. The sensor control device 52 adds sensor data information or the like to signals output from the near-infrared sensor 34, the RGB sensor 35, and the potential sensor 36 and transmits them to the cloud system 54. The culture control device 51 and the sensor control device 52 may transmit various pieces of data to the cloud system 54 at predetermined time intervals during a culture period.

In step S7, the database 40 stores various pieces of data received from the culture control device 51 and the sensor control device 52. The cloud system 54 may start subsequent processing in an event-driven manner when various pieces of data arrive from the culture control device 51 and the sensor control device 52.

In step S8, the data extraction system 1 reads various pieces of data as input signals from the database 40 and extracts extracted data. Detailed processing of the data extraction system 1 will be described later. The data extraction system 1 stores the extracted data in the database 40.

In step S9, the data analysis system 55 performs machine learning based on the extracted data as non-real-time processing. The dimension reduction unit 56 compresses dimensions of the extracted data by, for example, principal component analysis or the like. The explanatory variable extraction unit 57 determines an explanatory variable from a dimension-compressed signal based on correlation with an objective variable in machine learning, regression analysis, or the like. The machine learning unit 58 performs machine learning using the determined explanatory variable to create machine learning models such as a prediction model for predicting lactic acid amount, glucose amount, product production amount, and the like, and a control model for controlling culture.

The processes of steps S1 to S9 may be repeatedly executed. For example, the bioproduction system 50 may collect data for each pattern of parameters and create a machine learning model.

In step S10, the cloud system 54 performs culture prediction as real-time processing. For example, the machine learning unit 58 performs culture prediction using the prediction model.

In step S11, the cloud system 54 performs culture control as real-time processing. For example, the machine learning unit 58 creates a control variable for controlling the culture control device 51 using a result of culture prediction and the control model. The data analysis system 55 transmits the control variable to the culture control device 51.

In step S12, the culture control device 51 controls an alkali addition amount for controlling pH, an oxygen injection amount for controlling DO, temperature, Agit, and the like in response to the control variable received from the cloud system 54. By reflecting the result in the culture tank 20, automatic control of culture is performed.

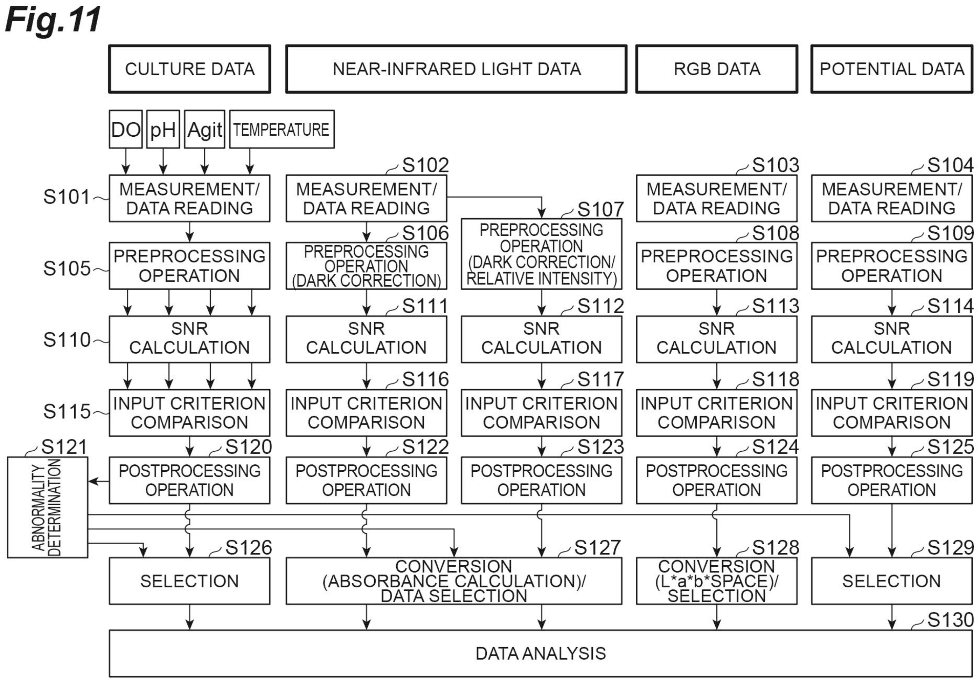

An example of operation of the data extraction system 1 will be described with reference to FIG. 11. FIG. 11 is a diagram showing an example of operation of the data extraction system 1. In FIG. 11, an example of measuring culture data, near-infrared light data, RGB data, and potential data will be described. In one example, the culture data are DO, pH, Agit, and temperature. Hereinafter, description will be made on the assumption that the setting unit 7 has already set the input criterion.

In step S101, a plurality of sensors 30 (for example, the pH sensor 31, the DO sensor, the torque sensor, and the temperature sensor 33) transmit signals indicating measurement results of DO, pH, Agit, and temperature to the culture control device 51. The culture control device 51 adds sensor data information or the like of the pH sensor 31, the DO sensor, the torque sensor, and the temperature sensor 33 to the signals indicating the measurement results and transmits them to the cloud system 54. The database 40 stores the signals (culture data) received from the culture control device 51. The input unit 2 of the data extraction system 1 acquires the culture data as an input signal by reading the culture data from the database.

In step S102, the near-infrared sensor 34 transmits a signal indicating a measurement result of an optical spectrum to the sensor control device 52. The sensor control device 52 adds sensor data information or the like of the near-infrared sensor 34 to the signal indicating the measurement result and transmits it to the cloud system 54. The database 40 stores the signal (near-infrared light data) received from the sensor control device 52. The input unit 2 of the data extraction system 1 acquires the near-infrared light data as an input signal by reading the near-infrared light data from the database 40.

In step S103, the RGB sensor 35 transmits a signal indicating a measurement result of RGB to the sensor control device 52. The sensor control device 52 adds sensor data information of the RGB sensor 35 to the signal indicating the measurement result and transmits it to the cloud system 54. The database 40 stores the signal (RGB data) received from the sensor control device 52. The input unit 2 of the data extraction system 1 acquires the RGB data as an input signal by reading the RGB data from the database 40.

In step S104, the potential sensor 36 transmits a signal indicating a measurement result of potential to the sensor control device 52. The sensor control device 52 adds sensor data information of the potential sensor 36 to the signal indicating the measurement result and transmits it to the cloud system 54. The database 40 stores the signal (potential data) received from the sensor control device 52. The input unit 2 of the data extraction system 1 acquires the potential data as an input signal by reading the potential data from the database 40.

In step S105, the preprocessing unit 3 performs a preprocessing operation such as gain adjustment and calibration on the input signal indicating the culture data.

In step S106, the preprocessing unit 3 performs dark correction on the input signal indicating the near-infrared light data.

In step S107, the preprocessing unit 3 performs dark correction and relative intensity correction on the input signal that is the near-infrared light data.

In step S108, the preprocessing unit 3 performs a preprocessing operation such as gain adjustment and calibration on the input signal indicating the RGB data.

In step S109, the preprocessing unit 3 performs a preprocessing operation such as gain adjustment and calibration on the input signal indicating the potential data.

In step S110, the preprocessing unit 3 generates a preprocessed signal related to the culture data by further calculating SNR using a signal obtained by performing the preprocessing operation on the input signal indicating the culture data.

In step S111, the preprocessing unit 3 generates a first preprocessed signal related to the near-infrared light data by further calculating SNR using a signal on which dark correction has been performed on the input signal indicating the near-infrared light data.

In step S112, the preprocessing unit 3 generates a second preprocessed signal related to the near-infrared light data by further calculating SNR using a signal on which dark correction and relative intensity correction have been performed on the input signal indicating the near-infrared light data.

In step S113, the preprocessing unit 3 generates a preprocessed signal related to the RGB data by further calculating SNR using a signal on which the preprocessing operation has been performed on the input signal indicating the RGB data.

In step S114, the preprocessing unit 3 generates a preprocessed signal related to the potential data by further calculating SNR using a signal on which the preprocessing operation has been performed on the input signal indicating the potential data.

A calculation method of SNR in steps S110 to S114 is not limited. In one example, the preprocessing unit 3 may measure in advance a noise level (N) that each of the sensors 30 has for each range, and calculate SNR [dB] by 20log(S/N) using a signal level (S). In another example, the preprocessing unit 3 may obtain a signal (S1) obtained by removing noise from an actual signal level (S), extract noise (N) by subtracting the signal (S1) obtained by removing noise from the actual signal level (S), and calculate SNR [dB] by 20log(S1/N).

In step S115, the comparison unit 8 compares the preprocessed signal related to the culture data with an input criterion corresponding to the signal to generate a comparison result related to the culture data.

In step S116, the comparison unit 8 compares the first preprocessed signal related to the near-infrared light data with a first input criterion corresponding to the signal to generate a first comparison result related to the near-infrared light data.

In step S117, the comparison unit 8 compares the second preprocessed signal related to the near-infrared light data with a second input criterion corresponding to the signal to generate a second comparison result related to the near-infrared light data.

In step S118, the comparison unit 8 compares the preprocessed signal related to the RGB data with an input criterion corresponding to the signal to generate a comparison result related to the RGB data.

In step S119, the comparison unit 8 compares the preprocessed signal related to the potential data with an input criterion corresponding to the signal to generate a comparison result related to the potential data.

In step S120, the postprocessing unit 4 performs a postprocessing operation for acquiring arithmetic mean or moving average on the preprocessed signal when the preprocessed signal related to the culture data does not satisfy the input criterion corresponding to the signal, to generate a postprocessed signal related to the culture data. When the preprocessed signal related to the culture data satisfies the input criterion corresponding to the signal, the postprocessing unit 4 does not perform the postprocessing operation.

In step S121, the abnormality determination unit 9 determines abnormality of culture based on the input signal. The abnormality determination unit 9 may detect abnormality based on the preprocessed signal related to the culture data or the postprocessed signal related to the culture data. The processing of step S121 may be performed before step S120.

The abnormality determination unit 9 may detect abnormality of the culture medium based on a change (decrease) in dissolved oxygen amount. For example, the abnormality determination unit 9 may detect a rapid decrease in DO by comparing fluctuation of DO per predetermined time with a predetermined threshold value or the like. The rapid decrease in DO may indicate that solidification of the culture medium is occurring. However, when Agit is at low rotation, solidification of the culture medium does not necessarily occur, and therefore the abnormality determination unit 9 may compare Agit with a threshold value corresponding to Agit.

The abnormality determination unit 9 may detect abnormality of electrode potential based on correlation between dissolved oxygen amount or pH and electrode potential. The abnormality determination unit 9 may monitor correlation between DO or pH and electrode potential, and determine that electrode potential that deviates from the correlation is in an abnormal state. For example, as a value of DO becomes smaller (decreases) or as a value of pH becomes larger (increases), it indicates that the culture medium is changing to alkaline. In potential measurement, there is a correlation in which electrode potential tends to decrease as the culture medium changes to alkaline. The abnormality determination unit 9 may determine that electrode potential is in an abnormal state when it makes a movement different from such correlation.

When the abnormality determination unit 9 detects abnormality, it may perform, as processing at the time of abnormality, sending of an error to the control terminal 53 or the like, or control of the sensor 30 or the like. The abnormality determination unit 9 replaces all measurement data with None data as processing at the time of abnormality. For example, the abnormality determination unit 9 replaces the culture data, the near-infrared light data, the RGB data, and the potential data with None data, respectively.

In step S122, the postprocessing unit 4 performs a postprocessing operation for acquiring arithmetic mean or moving average on the signal when the first preprocessed signal related to the near-infrared light data does not satisfy the first input criterion corresponding to the signal, to generate a first postprocessed signal related to the near-infrared light data. When the first preprocessed signal related to the near-infrared light data satisfies the first input criterion corresponding to the signal, the postprocessing unit 4 does not perform the postprocessing operation.

In step S123, the postprocessing unit 4 performs a postprocessing operation for acquiring arithmetic mean or moving average on the signal when the second preprocessed signal related to the near-infrared light data does not satisfy the input criterion corresponding to the signal, to generate a second postprocessed signal related to the near-infrared light data. When the second preprocessed signal related to the near-infrared light data satisfies the second input criterion corresponding to the signal, the postprocessing unit 4 does not perform the postprocessing operation.

In step S124, the postprocessing unit 4 performs a postprocessing operation for acquiring arithmetic mean or moving average on the preprocessed signal when the preprocessed signal related to the RGB data does not satisfy the input criterion corresponding to the signal, to generate a postprocessed signal related to the RGB data. When the preprocessed signal related to the RGB data satisfies the input criterion corresponding to the signal, the postprocessing unit 4 does not perform the postprocessing operation.

In step S125, the postprocessing unit 4 performs a postprocessing operation for acquiring arithmetic mean or moving average on the preprocessed signal when the preprocessed signal related to the potential data does not satisfy the input criterion corresponding to the signal, to generate a postprocessed signal related to the potential data. When the preprocessed signal related to the potential data satisfies the input criterion corresponding to the signal, the postprocessing unit 4 does not perform the postprocessing operation.

In one example, the input criterion is SNR. When capturing a change in the culture medium by the sensor 30, since a difference from an initial value is monitored, a signal level of the input signal is expected to be very small. If SNR of the input criterion is 10 dB and SNR of the preprocessed signal is 0 dB, the preprocessed signal does not satisfy the input criterion. When an input signal is obtained once per minute, the postprocessing unit 4 acquires arithmetic mean using input signals for 10 minutes (10 times). Thereby, 10 dB related to the comparison result can be eliminated.

In another example, when the SNR of the input criterion is 20 dB and SNR of the preprocessed signal is 0 dB, the preprocessed signal does not satisfy the input criterion. When an input signal is obtained once per minute, the postprocessing unit 4 acquires arithmetic mean using input signals for 10 minutes of 100 times. Thereby, 20 dB related to the comparison result can be eliminated. That is, acquisition of the input signal for the postprocessing operation requires 1000 minutes (approximately 16.7 hours). Here, if the signal level of the input signal becomes 10 times (difference from the initial value becomes 10 times) after 1 hour, it becomes possible to acquire extracted data of 20 dB in units of 10 minutes.

In step S126, when the preprocessed signal related to the culture data satisfies the input criterion corresponding to the signal, the extraction unit 5 extracts the preprocessed signal related to the culture data as extracted data related to the culture data. When the postprocessed signal related to the culture data satisfies the input criterion corresponding to the signal, the extraction unit 5 extracts the postprocessed signal related to the culture data as extracted data related to the culture data. When the postprocessed signal related to the culture data does not satisfy the input criterion corresponding to the signal, the extraction unit 5 removes the postprocessed signal related to the culture data. For example, the extraction unit 5 may remove the postprocessed signal related to the culture data by replacing the postprocessed signal related to the culture data with None data. The extraction unit 5 may extract None data as extracted data related to the culture data.

In step S127, when the first preprocessed signal related to the near-infrared light data satisfies the input criterion corresponding to the signal, the extraction unit 5 calculates absorbance using the first preprocessed signal related to the near-infrared light data. The extraction unit 5 extracts the absorbance as first extracted data related to the near-infrared light data. When the first postprocessed signal related to the near-infrared light data satisfies the input criterion corresponding to the signal, the extraction unit 5 calculates absorbance using the first postprocessed signal related to the near-infrared light data. The extraction unit 5 extracts the absorbance as first extracted data related to the near-infrared light data. When the first postprocessed signal related to the near-infrared light data does not satisfy the first input criterion corresponding to the signal, the extraction unit 5 removes the first postprocessed signal related to the near-infrared light data. For example, the extraction unit 5 may remove the first postprocessed signal related to the near-infrared light data by replacing the first postprocessed signal related to the near-infrared light data with None data. The extraction unit 5 may extract None data as first extracted data related to the near-infrared light data.

In step S127, when the second preprocessed signal related to the near-infrared light data satisfies the input criterion corresponding to the signal, the extraction unit 5 calculates absorbance using the second preprocessed signal related to the near-infrared light data. The extraction unit 5 extracts the absorbance as second extracted data related to the near-infrared light data. When the second postprocessed signal related to the near-infrared light data satisfies the input criterion corresponding to the signal, the extraction unit 5 calculates absorbance using the second postprocessed signal related to the near-infrared light data. The extraction unit 5 extracts the absorbance as second extracted data related to the near-infrared light data. When the second postprocessed signal related to the near-infrared light data does not satisfy the second input criterion corresponding to the signal, the extraction unit 5 removes the second postprocessed signal related to the near-infrared light data. For example, the extraction unit 5 may remove the second postprocessed signal related to the near-infrared light data by replacing the second postprocessed signal related to the near-infrared light data with None data. The extraction unit 5 may extract None data as second extracted data related to the near-infrared light data.

In step S128, when the preprocessed signal related to the RGB data satisfies the input criterion corresponding to the signal, the extraction unit 5 calculates a value of $\Delta E^*ab$ in an $L^*a^*b^*$ color space using the preprocessed signal related to the RGB data. The extraction unit 5 extracts the value of $\Delta E^*ab$ as extracted data related to the RGB data. When the postprocessed signal related to the RGB data satisfies the input criterion corresponding to the signal, the extraction unit 5 calculates a value of $\Delta E^*ab$ in an $L^*a^*b^*$ color space using the postprocessed signal related to the RGB data. The extraction unit 5 extracts the value of $\Delta E^*ab$ as extracted data related to the RGB data. When the postprocessed signal related to the RGB data does not satisfy the input criterion corresponding to the signal, the extraction unit 5 removes the postprocessed signal related to the RGB data. For example, the extraction unit 5 may remove the postprocessed signal related to the RGB data by replacing the postprocessed signal related to the RGB data with None data. The extraction unit 5 may extract None data as extracted data related to the RGB data.

In step S129, when the preprocessed signal related to the potential data satisfies the input criterion corresponding to the signal, the extraction unit 5 extracts the preprocessed signal related to the potential data as extracted data related to the potential data. When the postprocessed signal related to the potential data satisfies the input criterion corresponding to the signal, the extraction unit 5 extracts the postprocessed signal related to the potential data as extracted data related to the potential data. When the postprocessed signal related to the potential data does not satisfy the input criterion corresponding to the signal, the extraction unit 5 removes the postprocessed signal related to the potential data. For example, the extraction unit 5 may remove the postprocessed signal related to the potential data by replacing the postprocessed signal related to the potential data with None data. The extraction unit 5 may extract None data as extracted data related to the potential data.

In step S130, the database 40 stores each extracted data. Each extracted data stored in the database 40 can be used for data analysis. Each extracted data may be used for machine learning by the data analysis system 55 or the like. For example, when each extracted data is used for learning data in machine learning, efficiency regarding construction of a prediction model is improved. The abnormality determination unit 9 may determine that there is abnormality when there is an input signal, a preprocessed signal, or a postprocessed signal that deviates from the prediction model.

[Hardware Configuration]

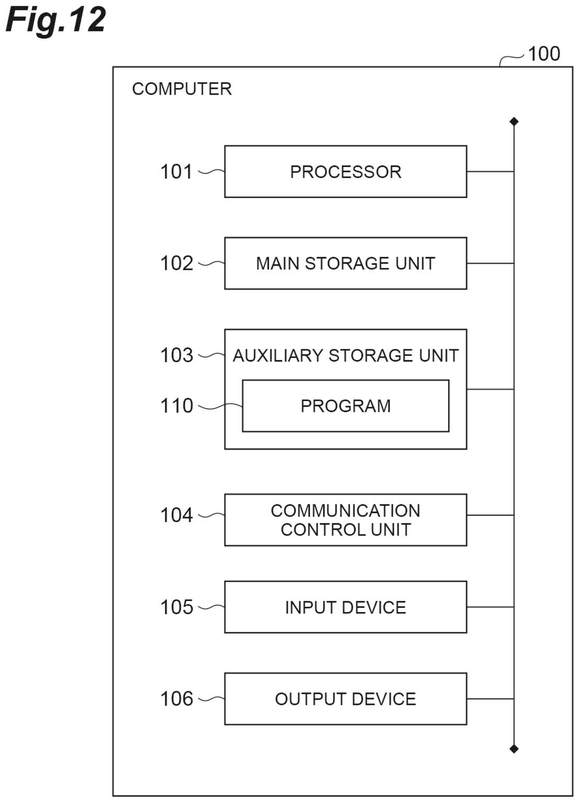

FIG. 12 is a diagram showing an example of hardware configuration related to the data extraction system 1. FIG. 12 shows a computer 100 that functions as the data extraction system 1. The computer 100 includes a processor 101, a main storage unit 102, an auxiliary storage unit 103, a communication control unit 104, an input device 105, and an output device 106. The data extraction system 1 is configured by one or more computers 100 configured by these hardware and software such as programs.

When the data extraction system 1 is configured by a plurality of computers 100, these computers 100 may be connected locally or may be connected via a communication network 59 such as the Internet or an intranet.

The processor 101 is a CPU (Central Processing Unit) that executes an operating system, application programs, and the like. The main storage unit 102 is configured by ROM (Read Only Memory) and RAM (Random Access Memory). The auxiliary storage unit 103 is a storage medium configured by a hard disk, flash memory, and the like. The auxiliary storage unit 103 generally stores a larger amount of data than the main storage unit 102. The communication control unit 104 is configured by a network card or a wireless communication module. At least a part of a communication function with other devices in the data extraction system 1 may be realized by the communication control unit 104. The input device 105 is configured by a keyboard, a mouse, a touch panel, a microphone for voice input, and the like. The output device 106 is configured by a display, a printer, and the like.

The auxiliary storage unit 103 stores in advance a program 110 (data extraction program) and data necessary for processing. The program 110 causes the computer 100 to execute each functional element of the data extraction system 1. By the program 110, for example, processing related to the above-described data extraction method is executed in the computer 100. For example, the program 110 is read by the processor 101 or the main storage unit 102, and operates at least one of the processor 101, the main storage unit 102, the auxiliary storage unit 103, the communication control unit 104, the input device 105, and the output device 106. For example, the program 110 reads and writes data in the main storage unit 102 and the auxiliary storage unit 103.

The program 110 may be provided after being recorded on a tangible storage medium such as a CD-ROM, a DVD-ROM, or a semiconductor memory. The program 110 may be provided as a data signal via the communication network 59.

As described above, the data extraction system 1 according to one aspect of the present disclosure includes: a setting unit 7 that sets an input criterion indicating performance required for data analysis; an input unit 2 that acquires an input signal indicating a measurement result of a measurement object in bioproduction; a preprocessing unit 3 that performs a preprocessing operation on the input signal for comparison with the input criterion to generate a preprocessed signal; a postprocessing unit 4 that performs a postprocessing operation using another signal indicating the measurement result of the measurement object on the preprocessed signal when the preprocessed signal does not satisfy the input criterion, to generate a postprocessed signal; and an extraction unit 5 that extracts the preprocessed signal as extracted data when the preprocessed signal satisfies the input criterion, and extracts the postprocessed signal as extracted data when the postprocessed signal satisfies the input criterion.

A data extraction method according to one aspect of the present disclosure is executed by a data extraction system including at least one processor. The data extraction method includes: a step of setting an input criterion indicating performance required for data analysis; a step of acquiring an input signal indicating a measurement result of a measurement object in bioproduction; a step of performing a preprocessing operation on the input signal for comparison with the input criterion to generate a preprocessed signal; a step of performing a postprocessing operation using another signal indicating the measurement result of the measurement object on the preprocessed signal when the preprocessed signal does not satisfy the input criterion, to generate a postprocessed signal; and a step of extracting the preprocessed signal as extracted data when the preprocessed signal satisfies the input criterion, and extracting the postprocessed signal as extracted data when the postprocessed signal satisfies the input criterion.

A data extraction program according to one aspect of the present disclosure causes a computer to execute: a step of setting an input criterion indicating performance required for data analysis; a step of acquiring an input signal indicating a measurement result of a measurement object in bioproduction; a step of performing a preprocessing operation on the input signal for comparison with the input criterion to generate a preprocessed signal; a step of performing a postprocessing operation using another signal indicating the measurement result of the measurement object on the preprocessed signal when the preprocessed signal does not satisfy the input criterion, to generate a postprocessed signal; and a step of extracting the preprocessed signal as extracted data when the preprocessed signal satisfies the input criterion, and extracting the postprocessed signal as extracted data when the postprocessed signal satisfies the input criterion.

In the data extraction system, the data extraction method, and the data extraction program according to one aspect of the present disclosure, the preprocessing operation is performed on the input signal to generate the preprocessed signal. Then, the preprocessed signal and the input criterion indicating performance required for data analysis are compared. When the preprocessed signal satisfies the input criterion, the preprocessed signal is extracted as the extracted data. On the other hand, when the preprocessed signal does not satisfy the input criterion, the postprocessing operation is performed on the preprocessed signal to generate the postprocessed signal. Then, the postprocessed signal and the input criterion are compared. When the postprocessed signal satisfies the input criterion, the postprocessed signal is extracted as the extracted data. Thereby, the extracted data whose quality is ensured can be extracted. As a result, the quality of data used for analysis can be improved.

The input signal is an optical spectrum measured by optical spectroscopic measurement in bioproduction. The preprocessing unit 3 generates the preprocessed signal by calculating a relative intensity with a signal level in a wavelength range that does not respond to a change in the culture medium and where a transmittance does not become zero. In conventional bioproduction, it has been performed to capture a change in a culture medium or the like while suppressing fluctuation noise by performing second-order differentiation on a spectrum signal in a wavelength direction. However, in this method, since the second-order differentiation is performed, it becomes difficult to directly capture a change in wavelength. In contrast, in the data extraction system of the present disclosure, relative intensity with a signal level in a wavelength range that does not respond to a change in the culture medium and where a transmittance does not become zero is calculated. By the preprocessed signal indicating relative intensity, fluctuation in signal level or the like caused by installation conditions of hardware or the like is eliminated. Thereby, detection accuracy of a target substance having low sensitivity is improved. As a result, the quality of data used for analysis can be improved.

The optical spectroscopic measurement is near-infrared spectroscopic measurement. The preprocessing unit 3 generates the preprocessed signal by calculating a relative intensity with a signal level in a wavelength range that does not respond to organic molecule fluctuation and where light intensity necessary for the near-infrared spectroscopic measurement is not affected by water absorption as the wavelength range. In this case, it becomes possible to detect a target substance having low sensitivity such as glucose concentration or lactic acid concentration.

The preprocessing unit 3 generates a first preprocessed signal for which relative intensity is calculated, and generates a second preprocessed signal for which relative intensity is not calculated. The setting unit 7 sets a first input criterion that is an input criterion corresponding to the first preprocessed signal, and sets a second input criterion that is an input criterion corresponding to the second preprocessed signal. The postprocessing unit 4 performs the postprocessing operation on the first preprocessed signal when the first preprocessed signal does not satisfy the first input criterion, to generate a first postprocessed signal. The extraction unit 5 extracts the first preprocessed signal as first extracted data when the first preprocessed signal satisfies the first input criterion, and extracts the first postprocessed signal as the first extracted data when the first postprocessed signal satisfies the first input criterion. The postprocessing unit 4 performs the postprocessing operation on the second preprocessed signal when the second preprocessed signal does not satisfy the second input criterion, to generate a second postprocessed signal. The extraction unit 5 extracts the second preprocessed signal as second extracted data when the second preprocessed signal satisfies the second input criterion, and extracts the second postprocessed signal as the second extracted data when the second postprocessed signal satisfies the second input criterion. In this case, extracted data based on the first preprocessed signal for which relative intensity is not calculated, and second extracted data based on the second preprocessed signal for which relative intensity is calculated are extracted. That is, data obtained by performing different preprocessing operations on the same signal is extracted. The first extracted data and the second extracted data can be used according to a purpose of data analysis when substances to be grasped as a change in the culture medium are different. Thereby, convenience of data analysis is improved.

The input criterion is a signal-to-noise ratio. By adopting SNR as the input criterion, unnecessary noise components are removed. As a result, efficiency and accuracy of data analysis are improved.

The postprocessing unit 4 performs arithmetic mean or moving average as the postprocessing operation. In this case, necessary accuracy of the postprocessed signal is improved.

The setting unit 7 further sets another input criterion different from the input criterion. The postprocessing unit 4 performs the postprocessing operation on the preprocessed signal when the preprocessed signal does not satisfy the other input criterion, to further generate another postprocessed signal. The extraction unit 5 further extracts the preprocessed signal as other extracted data when the preprocessed signal satisfies the other input criterion, and further extracts the postprocessed signal as other extracted data when the other postprocessed signal satisfies the other input criterion. In this case, extracted data based on the input criterion and other extracted data based on the other input criterion are extracted. That is, data having different properties is extracted by setting different input criteria for the same signal.

An abnormality determination unit 9 is provided that determines abnormality based on the input signal, the preprocessed signal, or the postprocessed signal, and invalidates the extracted data when abnormality is detected. Thereby, the extracted data whose quality is ensured can be extracted. As a result, the quality of data used for analysis can be improved.

The abnormality determination unit 9 detects abnormality of the culture medium based on a decrease in dissolved oxygen amount. In this case, it is possible to accurately detect that the culture medium is solidified.

The abnormality determination unit 9 detects abnormality of electrode potential based on correlation between dissolved oxygen amount or pH and electrode potential. In this case, it is possible to accurately detect abnormality in which the culture medium is changing to alkaline.

A data extraction system according to one aspect of the present disclosure acquires an optical spectrum measured by optical spectroscopic measurement in bioproduction, and outputs a signal obtained by calculating relative intensity with a signal level of the optical spectrum in a wavelength range that does not respond to a change in the culture medium and where a transmittance does not become zero with respect to the optical spectrum.

In the data extraction system according to one aspect of the present disclosure, relative intensity with a signal level in a wavelength range that does not respond to a change in the culture medium and where a transmittance does not become zero is calculated. By the signal indicating relative intensity, fluctuation in signal level or the like caused by installation conditions of hardware or the like is eliminated. Thereby, detection accuracy of a target substance having low sensitivity is improved. As a result, the quality of data used for analysis can be improved.

[Modification]

The present disclosure is not necessarily limited to the above-described embodiment, and various modifications are possible without departing from the gist thereof.

In bioproduction, the evaluation index may be constant or may change. For example, the evaluation index may change according to a substance to be evaluated during a period from start to end of culture. In one example, the evaluation index may correspond to substance A in an early stage of culture, correspond to substance B in a middle stage of culture, and correspond to substance C in a later stage of culture. In this case, the data extraction system extracts extracted data focused on for each process.

[Supplementary Notes]

Hereinafter, the gist of the present disclosure is shown.

[1] A data extraction system including:

a setting unit that sets an input criterion indicating performance required for data analysis;
an input unit that acquires an input signal indicating a measurement result of a measurement object in bioproduction;
a preprocessing unit that performs a preprocessing operation on the input signal for comparison with the input criterion to generate a preprocessed signal;
a postprocessing unit that performs a postprocessing operation using another signal indicating the measurement result of the measurement object on the preprocessed signal when the preprocessed signal does not satisfy the input criterion, to generate a postprocessed signal; and
an extraction unit that extracts the preprocessed signal as extracted data when the preprocessed signal satisfies the input criterion, and extracts the postprocessed signal as extracted data when the postprocessed signal satisfies the input criterion.

[2] The data extraction system according to [1], wherein
the input signal is an optical spectrum measured by optical spectroscopic measurement in bioproduction, and
the preprocessing unit generates the preprocessed signal by calculating a relative intensity with a signal level in a wavelength range that does not respond to a change in a culture medium and where a transmittance does not become zero.

[3] The data extraction system according to [2], wherein
the optical spectroscopic measurement is near-infrared spectroscopic measurement, and
the preprocessing unit generates the preprocessed signal by calculating a relative intensity with a signal level in a wavelength range that does not respond to organic molecule fluctuation and where light intensity necessary for the near-infrared spectroscopic measurement is not affected by water absorption as the wavelength range.

[4] The data extraction system according to [3], wherein the preprocessing unit generates a first preprocessed signal for which the relative intensity is not calculated, and generates a second preprocessed signal for which the relative intensity is calculated,

the setting unit sets a first input criterion that is an input criterion corresponding to the first preprocessed signal, and sets a second input criterion that is an input criterion corresponding to the second preprocessed signal,
the postprocessing unit performs the postprocessing operation on the first preprocessed signal when the first preprocessed signal does not satisfy the first input criterion, to generate a first postprocessed signal,
the extraction unit extracts the first preprocessed signal as first extracted data when the first preprocessed signal satisfies the first input criterion, and extracts the first postprocessed signal as first extracted data when the first postprocessed signal satisfies the first input criterion,
the postprocessing unit performs the postprocessing operation on the second preprocessed signal when the second preprocessed signal does not satisfy the second input criterion, to generate a second postprocessed signal, and
the extraction unit extracts the second preprocessed signal as second extracted data when the second preprocessed signal satisfies the second input criterion, and extracts the second postprocessed signal as second extracted data when the second postprocessed signal satisfies the second input criterion.

[5] The data extraction system according to any one of [1] to [4], wherein the input criterion is a signal-to-noise ratio.
[6] The data extraction system according to any one of [1] to [5], wherein the postprocessing unit performs arithmetic mean or moving average as the postprocessing operation.
[7] The data extraction system according to any one of [1] to [6], wherein
the setting unit further sets another input criterion different from the input criterion,

the postprocessing unit performs the postprocessing operation on the preprocessed signal when the preprocessed signal does not satisfy the other input criterion, to further generate another postprocessed signal, and
the extraction unit further extracts the preprocessed signal as other extracted data when the preprocessed signal satisfies the other input criterion, and further extracts the postprocessed signal as other extracted data when the other postprocessed signal satisfies the other input criterion.

[8] The data extraction system according to any one of [1] to [7], further including an abnormality determination unit that determines abnormality based on the input signal, the preprocessed signal, or the postprocessed signal, and invalidates the extracted data when abnormality is detected.

[9] The data extraction system according to [8], wherein the abnormality determination unit detects abnormality of a culture medium based on a decrease in dissolved oxygen amount.

[10] The data extraction system according to [8], wherein the abnormality determination unit detects abnormality of electrode potential based on correlation between dissolved oxygen amount or pH and electrode potential.

[11] A data extraction method executed by a data extraction system including at least one processor, the data extraction method including:

a step of setting an input criterion indicating performance required for data analysis;
a step of acquiring an input signal indicating a measurement result of a measurement object in bioproduction;
a step of performing a preprocessing operation on the input signal for comparison with the input criterion to generate a preprocessed signal;
a step of performing a postprocessing operation using another signal indicating the measurement result of the measurement object on the preprocessed signal when the preprocessed signal does not satisfy the input criterion, to generate a postprocessed signal; and
a step of extracting the preprocessed signal as extracted data when the preprocessed signal satisfies the input criterion, and extracting the postprocessed signal as extracted data when the postprocessed signal satisfies the input criterion.

[12] A data extraction program for causing a computer to execute:
a step of setting an input criterion indicating performance required for data analysis;

a step of acquiring an input signal indicating a measurement result of a measurement object in bioproduction;
a step of performing a preprocessing operation on the input signal for comparison with the input criterion to generate a preprocessed signal;
a step of performing a postprocessing operation using another signal indicating the measurement result of the measurement object on the preprocessed signal when the preprocessed signal does not satisfy the input criterion, to generate a postprocessed signal; and
a step of extracting the preprocessed signal as extracted data when the preprocessed signal satisfies the input criterion, and extracting the postprocessed signal as extracted data when the postprocessed signal satisfies the input criterion.

[13] A data extraction system that acquires an optical spectrum measured by optical spectroscopic measurement in bioproduction, and
outputs a signal obtained by calculating relative intensity with a signal level of the optical spectrum in a wavelength range that does not respond to a change in a culture medium and where a transmittance does not become zero with respect to the optical spectrum.

**Reference Signs List**

1 Data extraction system, 2 Input unit, 3 Preprocessing unit, 4 Postprocessing unit, 5 Extraction unit, 6 Control unit 7 Setting unit, 8 Comparison unit, 9 Abnormality determination unit, 10 Control block, 20 Culture tank, 21 Light source, 22 Flow cell, 23 Optical spectrometer, 24 Graph, 30 Sensor, 40 Database, 50 Bioproduction system, 51 Culture control device, 52 Sensor control device, 53 Control terminal, 54 Cloud system, 55 Data analysis system, 56 Dimension reduction unit, 57 Explanatory variable extraction unit, 58 Machine learning unit, 59 Communication network, 100 Computer, 101 Processor, 102 Main storage unit, 103 Auxiliary storage unit, 104 Communication control unit, 105 Input device, 106 Output device, 110 Program

**Claims**

1. A data extraction system comprising:

a setting unit that sets an input criterion indicating performance required for data analysis;
an input unit that acquires an input signal indicating a measurement result of a measurement object in bioproduction;
a preprocessing unit that performs a preprocessing operation on the input signal for comparison with the input criterion to generate a preprocessed signal;
a postprocessing unit that performs a postprocessing operation using another signal indicating the measurement result of the measurement object on the preprocessed signal when the preprocessed signal does not satisfy the input criterion, to generate a postprocessed signal; and
an extraction unit that extracts the preprocessed signal as extracted data when the preprocessed signal satisfies the input criterion, and extracts the postprocessed signal as extracted data when the postprocessed signal satisfies the input criterion.

2. The data extraction system according to claim 1, wherein the input signal is an optical spectrum measured by optical spectroscopic measurement in bioproduction, and
the preprocessing unit generates the preprocessed signal by calculating a relative intensity with a signal level in a wavelength range that does not respond to a change in a culture medium and where a transmittance does not become zero.

3. The data extraction system according to claim 2, wherein the optical spectroscopic measurement is near-infrared spectroscopic measurement, and the preprocessing unit generates the preprocessed signal by calculating a relative intensity with a signal level in a wavelength range that does not respond to organic molecule fluctuation and where light intensity necessary for the near-infrared spectroscopic measurement is not affected by water absorption as the wavelength range.

4. The data extraction system according to claim 3, wherein the preprocessing unit generates a first preprocessed signal for which the relative intensity is not calculated, and generates a second preprocessed signal for which the relative intensity is calculated,

the setting unit sets a first input criterion that is an input criterion corresponding to the first preprocessed signal, and sets a second input criterion that is an input criterion corresponding to the second preprocessed signal,
the postprocessing unit performs the postprocessing operation on the first preprocessed signal when the first preprocessed signal does not satisfy the first input criterion, to generate a first postprocessed signal,
the extraction unit extracts the first preprocessed signal as first extracted data when the first preprocessed signal satisfies the first input criterion, and extracts the first postprocessed signal as first extracted data when the first postprocessed signal satisfies the first input criterion,
the postprocessing unit performs the postprocessing operation on the second preprocessed signal when the second preprocessed signal does not satisfy the second input criterion, to generate a second postprocessed signal, and
the extraction unit extracts the second preprocessed signal as second extracted data when the second preprocessed signal satisfies the second input criterion, and extracts the second postprocessed signal as second extracted data when the second postprocessed signal satisfies the second input criterion.

5. The data extraction system according to claim 4, wherein the input criterion is a signal-to-noise ratio.

6. The data extraction system according to claim 5, wherein the postprocessing unit performs arithmetic mean or moving average as the postprocessing operation.

7. The data extraction system according to claim 1, wherein the setting unit further sets another input criterion different from the input criterion,

the postprocessing unit performs the postprocessing operation on the preprocessed signal when the preprocessed signal does not satisfy the other input criterion, to further generate another postprocessed signal, and
the extraction unit further extracts the preprocessed signal as other extracted data when the preprocessed signal satisfies the other input criterion, and further extracts the postprocessed signal as other extracted data when the other postprocessed signal satisfies the other input criterion.

8. The data extraction system according to claim 1, further comprising an abnormality determination unit that determines abnormality based on the input signal, the preprocessed signal, or the postprocessed signal, and invalidates the extracted data when abnormality is detected.

9. The data extraction system according to claim 8, wherein the abnormality determination unit detects abnormality of a culture medium based on a decrease in dissolved oxygen amount.

10. The data extraction system according to claim 8, wherein the abnormality determination unit detects abnormality of electrode potential based on correlation between dissolved oxygen amount or pH and electrode potential.

11. A data extraction method executed by a data extraction system including at least one processor, the data extraction method comprising:

a step of setting an input criterion indicating performance required for data analysis;
a step of acquiring an input signal indicating a measurement result of a measurement object in bioproduction;
a step of performing a preprocessing operation on the input signal for comparison with the input criterion to generate a preprocessed signal;
a step of performing a postprocessing operation using another signal indicating the measurement result of the measurement object on the preprocessed signal when the preprocessed signal does not satisfy the input criterion, to generate a postprocessed signal; and

a step of extracting the preprocessed signal as extracted data when the preprocessed signal satisfies the input criterion, and extracting the postprocessed signal as extracted data when the postprocessed signal satisfies the input criterion.

**12.** A data extraction program for causing a computer to execute:

a step of setting an input criterion indicating performance required for data analysis;

a step of acquiring an input signal indicating a measurement result of a measurement object in bioproduction;

a step of performing a preprocessing operation on the input signal for comparison with the input criterion to generate a preprocessed signal;

a step of performing a postprocessing operation using another signal indicating the measurement result of the measurement object on the preprocessed signal when the preprocessed signal does not satisfy the input criterion, to generate a postprocessed signal; and

a step of extracting the preprocessed signal as extracted data when the preprocessed signal satisfies the input criterion, and extracting the postprocessed signal as extracted data when the postprocessed signal satisfies the input criterion.

**13.** A data extraction system that acquires an optical spectrum measured by optical spectroscopic measurement in bioproduction, and

outputs a signal obtained by calculating relative intensity with a signal level of the optical spectrum in a wavelength range that does not respond to a change in a culture medium and where a transmittance does not become zero with respect to the optical spectrum.

# Fig.1

# Fig.2

**Fig.3**

EP 4 741 487 A1

# Fig.4

(a) SIGNAL OUTPUT (TRANSMITTED LIGHT INTENSITY)

(b) SIGNAL OUTPUT-Ndc

(c) SIGNAL OUTPUT-Ndc   RELATIVE INTENSITY (/1300nm)

**Fig.5**

EP 4 741 487 A1

**Fig.6**

| INPUT SIGNAL | PREPROCESSING OPERATION | EVALUATION INDEX | POSTPROCESSING OPERATION | DATA CONVERSION/ SELECTION | DATABASE |
|---|---|---|---|---|---|

e4 — EVALUATION INDEX
e5 — EVALUATION INDEX
e6 — EVALUATION INDEX
e7 — EVALUATION INDEX

x7 — INPUT SIGNAL

f6 — PREPROCESSED SIGNAL / DARK CORRECTION
c4 — INPUT CRITERION
p6 — POSTPROCESSED SIGNAL
CONVERSION/ SELECTION
d4 — EXTRACTED DATA

f7 — PREPROCESSED SIGNAL / DARK CORRECTION / RELATIVE INTENSITY CORRECTION
c5 — INPUT CRITERION
p7 — POSTPROCESSED SIGNAL
CONVERSION/ SELECTION
d5 — EXTRACTED DATA

f8 — PREPROCESSED SIGNAL / DARK CORRECTION
c6 — INPUT CRITERION
p8 — POSTPROCESSED SIGNAL
CONVERSION/ SELECTION
d6 — EXTRACTED DATA

f9 — PREPROCESSED SIGNAL / DARK CORRECTION / RELATIVE INTENSITY CORRECTION
c7 — INPUT CRITERION
p9 — POSTPROCESSED SIGNAL
CONVERSION/ SELECTION
d7 — EXTRACTED DATA

EP 4 741 487 A1

Fig.7

# Fig.8

(a)

(b)

(c)

(d)

EP 4 741 487 A1

**Fig.9**

**Fig.10**

EP 4 741 487 A1

# Fig.11

CULTURE DATA | NEAR-INFRARED LIGHT DATA | RGB DATA | POTENTIAL DATA

DO | pH | Agit | TEMPERATURE

S101 — MEASUREMENT/DATA READING
S102 — MEASUREMENT/DATA READING
S103 — MEASUREMENT/DATA READING
S104 — MEASUREMENT/DATA READING

S105 — PREPROCESSING OPERATION
S106 — PREPROCESSING OPERATION (DARK CORRECTION)
S107 — PREPROCESSING OPERATION (DARK CORRECTION/RELATIVE INTENSITY)
S108 — PREPROCESSING OPERATION
S109 — PREPROCESSING OPERATION

S110 — SNR CALCULATION
S111 — SNR CALCULATION
S112 — SNR CALCULATION
S113 — SNR CALCULATION
S114 — SNR CALCULATION

S115 — INPUT CRITERION COMPARISON
S116 — INPUT CRITERION COMPARISON
S117 — INPUT CRITERION COMPARISON
S118 — INPUT CRITERION COMPARISON
S119 — INPUT CRITERION COMPARISON

S121 — ABNORMALITY DETERMINATION

S120 — POSTPROCESSING OPERATION
S122 — POSTPROCESSING OPERATION
S123 — POSTPROCESSING OPERATION
S124 — POSTPROCESSING OPERATION
S125 — POSTPROCESSING OPERATION

S126 — SELECTION
S127 — CONVERSION (ABSORBANCE CALCULATION)/DATA SELECTION
S128 — CONVERSION (L*a*b*SPACE)/SELECTION
S129 — SELECTION

S130 — DATA ANALYSIS

EP 4 741 487 A1

32

# Fig.12

# EP 4 741 487 A1

<table>
<tr><td colspan="2"><strong>INTERNATIONAL SEARCH REPORT</strong></td><td>International application No.<br><strong>PCT/JP2024/024986</strong></td></tr>
</table>

| A. | CLASSIFICATION OF SUBJECT MATTER |
|---|---|

*C12M 1/34*(2006.01)i; *G01N 21/80*(2006.01)i; *G01N 21/359*(2014.01)i
FI:   C12M1/34 Z; G01N21/359; G01N21/80

According to International Patent Classification (IPC) or to both national classification and IPC

| B. | FIELDS SEARCHED |
|---|---|

Minimum documentation searched (classification system followed by classification symbols)

C12M1/34; G01N21/80; G01N21/359

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2013-544353 A (F. HOFFMANN-LA ROCHE AG) 12 December 2013 (2013-12-12)<br>entire text, all drawings | 1-13 |
| A | US 2021/0372932 A1 (KAISER OPTICAL SYSTEMS INC.) 02 December 2021 (2021-12-02)<br>entire text, all drawings | 1-13 |
| A | 坂井絵美　他, 近赤外分光法による大豆発酵食品「テンペ」の発酵状態評価の可能性, 名古屋文理大学紀要, 2013, vol. 13, pp. 19-26, ISSN: 2433-5517, (SAKAI, Emi et al., The feasibility of the fermentation condition evaluation of "tempeh" which is soybean fermented food by NIR Spectroscopy, Journal of Nagoya Bunri University)<br>entire text, all drawings | 1-13 |
| A | NESPECA, M. G. et al. Determination of alcohols and volatile organic acids in anaerobic bioreactors for H2 production by near infrared spectroscopy. International Journal of Hydrogen Energy. 2017, vol. 42, pp. 20480-20493, ISSN: 0360-3199<br>entire text, all drawings | 1-13 |

☑ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 September 2024** | **01 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

34

**INTERNATIONAL SEARCH REPORT**

| International application No. |
| --- |
| **PCT/JP2024/024986** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2008-203234 A (MATSUSHITA ELECTRIC WORKS, LTD.) 04 September 2008 (2008-09-04)<br>entire text, all drawings | 1-13 |

Form PCT/ISA/210 (second sheet) (July 2022)

**INTERNATIONAL SEARCH REPORT**
Information on patent family members

International application No.

**PCT/JP2024/024986**

| Patent document cited in search report | | | Publication date (day/month/year) | Patent family member(s) | | | Publication date (day/month/year) |
|---|---|---|---|---|---|---|---|
| JP | 2013-544353 | A | 12 December 2013 | US | 2014/0032127 | A1 | |
| | | | | entire text, all drawings | | | |
| | | | | WO | 2012/059520 | A1 | |
| | | | | EP | 2635892 | A1 | |
| | | | | CN | 103201616 | A | |
| | | | | KR | 10-2013-0079571 | A | |
| US | 2021/0372932 | A1 | 02 December 2021 | EP | 3865861 | A1 | |
| | | | | CN | 113252634 | A | |
| JP | 2008-203234 | A | 04 September 2008 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6977977 B

- JP 2022007236 A